(19) Europäisches Patentamt / European Patent Office / Office européen des brevets

(11) EP 3 484 612 B1

(12) **EUROPÄISCHE PATENTSCHRIFT**

(45) Veröffentlichungstag und Bekanntmachung des Hinweises auf die Patenterteilung:
**08.12.2021 Patentblatt 2021/49**

(21) Anmeldenummer: **18700896.6**

(22) Anmeldetag: **16.01.2018**

(51) Int Cl.:
***B01J 20/24*** (2006.01) ***B01J 20/26*** (2006.01)
***B01D 15/38*** (2006.01) ***B01J 20/32*** (2006.01)
***C07K 1/22*** (2006.01) ***B01J 20/28*** (2006.01)
***B01J 20/286*** (2006.01)

(86) Internationale Anmeldenummer:
**PCT/EP2018/051016**

(87) Internationale Veröffentlichungsnummer:
**WO 2018/137975 (02.08.2018 Gazette 2018/31)**

(54) **CHROMATOGRAPHIEMEDIUM MIT GEBUNDENEN MIKROGLOBULI UND VERFAHREN ZU DESSEN HERSTELLUNG**

CHROMATOGRAPHY MEDIUM HAVING BONDED MICROGLOBULI AND METHOD FOR THE PRODUCTION THEREOF

SUPPORT CHROMATOGRAPHIQUE COMPRENANT DES MICROGLOBULES LIÉS ET SON PROCÉDÉ DE FABRICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.01.2017 DE 102017000631**

(43) Veröffentlichungstag der Anmeldung:
**22.05.2019 Patentblatt 2019/21**

(73) Patentinhaber: **Sartorius Stedim Biotech GmbH**
**37079 Göttingen (DE)**

(72) Erfinder:
- **LEY, Adrian**
  **37081 Göttingen (DE)**
- **TAFT, Florian**
  **37085 Göttingen (DE)**
- **SCHWELLENBACH, Jan**
  **37127 Dransfeld (DE)**
- **VILLAIN, Louis**
  **D-37079 Göttingen (DE)**

(74) Vertreter: **Vigand, Philippe**
**Novagraaf International SA**
**Chemin de l'Echo 3**
**1213 Onex - Genève (CH)**

(56) Entgegenhaltungen:
**WO-A1-93/25594**
**WO-A1-98/37949**
**DE-A1- 10 344 820**
**US-B1- 6 451 260**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 484 612 B1

Printed by Jouve, 75001 PARIS (FR)

## Beschreibung

Die vorliegende Erfindung betrifft ein Chromatographiemedium, das in der Affinitätschromatographie verwendet werden kann, sowie ein Verfahren zu dessen Herstellung.

Im Downstreamprozess ist die Protein A Affinitätschromatographie für die Aufreinigung von Antikörpern eine wichtige Methode aufgrund hoher Effizienz. In einem einzigen Schritt werden hohe Reinheitsgrade des Antikörpers erreicht, was diese Methode sowohl ökonomisch als auch ökologisch von anderen Aufreinigungsmethoden unterscheidet. Wie bei allen präparativchromatographischen Methoden ist die Produktivität eines chromatographischen Mediums von entscheidender Bedeutung. Insbesondere setzt sich diese aus dem Fluss, den das Medium zulässt (Permeabilität), und der dynamischen Bindungskapazität zusammen. Die bisher im Stand der Technik bekannten Chromatographiemedien zeigen jedoch entweder einen niedrigen Fluss bzw. eine geringe Permeabilität (z.B. Protein A Gelsäulen) oder eine niedrige dynamische Bindungskapazität (z.B. Protein A Membranadsorber).

Im Stand der Technik (SdT) sind Chromatographiemedien bekannt, in denen poröse Matrices derart modifiziert werden, dass sie ein makroporöses (un)-vernetztes (Hydro)-Gel in ihren Poren aufweisen (siehe beispielsweise US 8,652,849 B2, US 7,316,919 B2, US 8,133,840 B2, US 7,247,370 B2, EP 1 776 176 B1; nachfolgend als SdT-Dokumente 1 bis 5 bezeichnet). Nach der Definition des "Dictionary of Polymers", Katsuyoshi Nishinari, Progr. Colloid Polym. Sci. (2009) 136: 87-94, "Some Thoughts on the Definition of a Gel", und "Gels: Structures, Properties, and Functions: Fundamentals and Applications" vom Katsuyoshi Nishinari (Springer Verlag Berlin Heidelberg 2009) besteht ein Polymergel aus einem dreidimensional vernetzten Netzwerk und quellt in einem Lösungsmittel bis zu einem gewissen begrenzten Ausmaß, löst sich jedoch selbst in einem guten Lösungsmittel für das Polymer als solches nicht. Nach der Definition von "Römpp Online 2014" ist ein Hydrogel ein Wasser enthaltendes, aber wasserunlösliches Polymer, dessen Moleküle chemisch, z.B. durch kovalente oder ionische Bindungen, oder physikalisch, z.B. durch Verschlaufen der Polymerketten, zu einem dreidimensionalen Netzwerk verknüpft sind. Durch eingebaute hydrophile Polymerkomponenten quellen Hydrogele in Wasser unter beträchtlicher Volumenzunahme, ohne aber ihren stofflichen Zusammenhalt zu verlieren. WO 93/25594 A1 beschreibt Träger mit Azlacton-funktionellen Oberflächen, Adduktträger und Verfahren zu ihrer Herstellung.

Auch diese im Stand der Technik bekannten Chromatographiemedien zeigen jedoch entweder einen niedrigen Fluss bzw. eine geringe Permeabilität oder eine niedrige dynamische Bindungskapazität.

Der vorliegenden Erfindung liegt somit die Aufgabe zugrunde, ein Chromatographiemedium bereitzustellen, das sowohl einen hohen Fluss erlaubt als auch eine hohe dynamische Bindungskapazität aufweist.

Diese Aufgabe wird durch die in den Ansprüchen gekennzeichneten Ausführungsformen der vorliegenden Erfindung gelöst.

Insbesondere wird erfindungsgemäß ein Chromatographiemedium bereitgestellt,

umfassend
eine poröse Matrix; und
unporöse Mikroglobuli,
wobei die unporösen Mikroglobuli auf den inneren und äußeren Oberflächen der porösen Matrix kovalent gebunden sind,
wobei der durchschnittliche Radius der Mikroglobuli nicht mehr als 30% des durchschnittlichen Porendurchmessers der porösen Matrix beträgt, wobei der Porendurchmesser der porösen Matrix und der Radius der Mikroglobuli über Rasterelektronenmikroskopie bestimmt werden;
wobei der Pfropfgrad P des Chromatographiemediums von 25% bis 40% beträgt, wobei gilt:

$$P = \frac{m_m - m_0}{m_0} * 100\%$$

wobei $m_m$ die Masse des Chromatographiemediums ist und mo die Masse der porösen Matrix ohne Mikroglobuli ist, und
wobei der relative Pfropfgrad $P_{rel}$ des Chromatographiemediums höchstens 0,25 g/cm$^3$ beträgt, wobei gilt:

$$P_{rel} = \frac{P * \rho}{Por}$$

wobei P der Pfropfgrad des Chromatographiemediums in % ist, ρ die Dichte der porösen Matrix ohne Mikroglobuli in g/cm$^3$ ist, und Por die Porosität der porösen Matrix ohne Mikroglobuli in % ist,
wobei die unporösen Mikroglobuli im Wesentlichen kugelförmige Oligomore und/oder Polymere sind, die aus mindestens einem Monomer, ausgewählt aus der Gruppe, bestehend aus Glycidyl(meth)acrylat, substituierten oder

unsubstituierten Alkyl(meth)acrylaten und ihren Derivaten, Styrol und seinen Derivaten, 2-Vinyl-4,4-dimethylazlacton, substituierten oder unsubstituierten N-Alkyl(meth)acrylamiden und ihren Derivaten und substituierten oder unsubstituierten N,N'-Dialkyl(meth)acrylamiden und ihren Derivaten, aufgebaut sind, und
wobei die Oligomere und/oder Polymere miteinander und mit den inneren und äußeren Oberflächen der porösen Matrix über einen oder mehrere Vernetzer, ausgewählt aus der Gruppe, bestehend aus Ethylenglycoldimethacrylat, Trimethylpropantrimethacrylat, Divinylbenzol und N,N'-Methylenbisacrylamid, vernetzt sind.

Unter dem Begriff "poröse Matrix" wird im Sinne der vorliegenden Erfindung jegliches poröse Material verstanden, das für chromatographische Verfahren als stationäre Phase verwendet werden kann. Die poröse Matrix der vorliegenden Erfindung, die im erfindungsgemäßen Verfahren als "Ausgangsmatrix" bezeichnet wird, unterliegt keiner besonderen Einschränkung, solange die Matrix Poren aufweist. Die durchschnittliche Größe bzw. der durchschnittliche Durchmesser der Poren der Matrix unterliegt keiner besonderen Einschränkung und es kann beispielsweise eine Matrix mit einem durchschnittlichen Porendurchmesser von 0,01 bis 20 $\mu$m, bevorzugt von 0,1 bis 10 $\mu$m und mehr bevorzugt von 0,2 bis 4 $\mu$m, verwendet werden.

Der weitere Aufbau der Matrix unterliegt keiner besonderen Beschränkung.

Optional kann daher die poröse Matrix beispielsweise eine Membran (einschließlich Hohlfasermembranen), ein Vlies aus Filamenten, Splitfasern, Nanofasern oder Hohlfasern, Kurzschnittfasern, oder ein Monolith sein. Auch das Material der porösen Matrix unterliegt keiner besonderen Einschränkung, und es können beispielsweise Membranen oder Vliese aus Cellulose-Hydrat/-Ester/-Acetat, Cellulose-Nanofasern, Nylon-Nanofasern, Polyethylenterephthalat (PET), Poly(oxy-1,4-phenylsulfonyl-1,4-phenyl), Polyamiden, Polyestern, Polyvinylidenfluorid (PVDF), Polyethylen (PE) und/oder Polypropylen (PP) verwendet werden.

Es wird entweder eine poröse Matrix eingesetzt, die originär zur radikalischen Polymerisation befähigte Gruppen aufweist, oder eine poröse Matrix, bei der durch eine dem Fachmann bekannte Oberflächenmodifikation funktionelle Gruppen eingeführt wurden. Bekannte Oberflächenmodifikationen sind in diesem Zusammenhang beispielsweise eine Pfropfpolymerisation von Monomeren mit funktionellen Gruppen ("grafting-through"-Polymerisation), Aktivierung mittels Plasmabehandlung, e-Beam (Elektronenstrahlbehandlung), Gamma-Bestrahlung, Hydrolyse, Aminolyse, Oxidation, Reduktion, Umsetzung mit funktionellen Carbenen und/oder Nitrenen usw.. Alternativ kann die poröse Matrix auch ohne die Anwesenheit von zur radikalischen Polymerisation befähigten Gruppen verwendet werden.

Erfindungsgemäß sind auf den inneren und äußeren Oberflächen der porösen Matrix unporöse Mikroglobuli (Mikrokügelchen) kovalent gebunden, z.B. durch Aufpfropfung. Unter dem Begriff "unporöse Mikroglobuli" werden im Sinne der vorliegenden Erfindung im Wesentlichen kugelförmige Strukturen verstanden, d.h. das Verhältnis des Minimaldurchmessers zum Maximaldurchmessers eines Mikroglobulus liegt üblicherweise bevorzugt in einem Bereich von 1:2 bis 1:1. Der durchschnittliche Radius der Mikroglobuli beträgt erfindungsgemäß nicht mehr als 30% des durchschnittlichen Porendurchmessers der porösen Matrix (d.h. der porösen Matrix ohne gebundene Mikroglobuli). Somit weisen die Mikroglobuli bevorzugt einen durchschnittlichen Radius von maximal ungefähr 6 $\mu$m auf, mehr bevorzugt von maximal 3 $\mu$m und am meisten bevorzugt von maximal 1,2 $\mu$m. Erfindungsgemäß wird unter dem Durchmesser bzw. dem Radius eines Mikroglobulus bzw. einer Pore der Maximaldurchmesser bzw. Maximalradius des Mikroglobulus bzw. der Pore verstanden. Sowohl der Durchmesser bzw. der Radius der Poren der porösen Matrix als auch der Durchmesser bzw. der Radius der Mikroglobuli werden über Rasterelektronenmikroskopie (REM) bestimmt.

Die Mikroglobuli des erfindungsgemäßen Chromatographiemediums sind keine Gele oder Hydrogele, da sie in hydrophilen Lösungsmitteln, beispielsweise Wasser und Aceton, wenn überhaupt dann nur sehr geringfügig quellen. Damit unterscheiden sich die Mikroglobuli signifikant von den (Hydro)-Gelen der SdT-Dokumente 1 bis 5.

Durch die Ausbildung unporöser Mikroglobuli auf den inneren und äußeren Oberflächen der porösen Matrix wird aufgrund der im Wesentlichen kugelförmigen Struktur der unporösen Mikroglobuli die spezifische Oberfläche des Chromatographiemediums vorteilhafterweise im Vergleich zu einem Chromatographiemedium, das lediglich aus einer porösen Matrix aufgebaut ist, signifikant erhöht. Die signifikant erhöhte Oberfläche des Chromatographiemediums der vorliegenden Erfindung erlaubt vorteilhafterweise eine höhere Anzahl an chromatographisch aktiven Zentren, so dass das erfindungsgemäße Chromatographiemedium eine erhöhte dynamische Bindungskapazität aufweist. Es werden dabei unter dem Begriff "chromatographisch aktive Zentren" funktionelle Oberflächengruppen verstanden, die mit bestimmten Komponenten von Fluiden selektiv Bindungen eingehen können, wobei die chromatographisch aktiven Zentren bereits originär in den Mikroglobuli vorhanden sein, oder aber auch durch eine zusätzliche Modifizierung an den Mikroglobuli immobilisiert werden können.

Da der durchschnittliche Radius der Mikroglobuli erfindungsgemäß nicht mehr als 30% des durchschnittlichen Porendurchmesser der porösen Matrix beträgt, bleibt die Porenstruktur der porösen Matrix weitestgehend erhalten, so dass die Permeabilität des erfindungsgemäßen Chromatographiemediums im Vergleich zu einem Chromatographiemedium, das lediglich aus einer porösen Matrix aufgebaut ist, vorteilhafterweise wenig beeinträchtigt ist. Demzufolge erlaubt das erfindungsgemäße Chromatographiemedium vorteilhafterweise einen hohen Fluss (Permeabilität) und weist zudem eine hohe dynamische Bindungskapazität auf. Gemäß einer bevorzugten Ausführungsform der vorliegenden

Erfindung beträgt die Permeabilität des Chromatographiemediums mindestens 40% der Permeabilität der Ausgangsmatrix (d.h. der porösen Matrix des Chromatographiemediums ohne gebundene Mikroglobuli), bevorzugt mindestens 50%, mehr bevorzugt mindestens 60% und am meisten bevorzugt mindestens 70%.

Zudem erlauben die Mikroglobuli eine Optimierung der Porengröße der porösen Matrix im Hinblick auf eine affinitätschromatographische Anwendung, d.h. insbesondere können solche Poren der porösen Matrix optimiert werden, die ohne die unporösen Mikroglobuli eine zu große Porengröße aufweisen würden, so dass ein Zielmolekül im Chromatographieprozess diese Poren konvektiv durchströmen würde, ohne dabei Wechselwirkungen mit der Oberfläche zu zeigen.

Erfindungsgemäß sind die Mikroglobuli unporös, d.h. sie weisen keine Poren auf. Durch die unporösen Mikroglobuli entstehen vorteilhafterweise keine Poren, die nur diffusiv erreichbar sind. Dies minimiert eine Abhängigkeit der dynamischen Bindungskapazität von der Verweilzeit, da diese dann ausschließlich von der Diffusion des Zielmoleküls der Chromatographie zum chromatographisch aktiven Zentrum abhängt. Zudem wird dadurch die Zugänglichkeit zu den chromatographisch aktiven Zentren in einem dynamischen Trennprozess erhöht, so dass insbesondere der Verbrauch an nachträglich in das Chromatographiemedium eingeführten chromatographisch aktiven Zentren wie beispielsweise (Affinitäts)-Liganden minimiert werden kann, da die Liganden während der Immobilisierung (Einfügung) nicht in für das Zielmolekül der Chromatographie unzugänglichen Poren immobilisiert werden.

Erfindungsgemäß sind die unporösen Mikroglobuli im Wesentlichen kugelförmige Oligomere und/oder Polymere, insbesondere Oligomere und/oder Polymere, die aus mindestens einem Monomer, ausgewählt aus der Gruppe, bestehend aus Glycidyl(meth)acrylat, substituierten oder unsubstituierten Alkyl(meth)acrylaten und ihren Derivaten (besonders bevorzugt Methyl(meth)acrylat, Butyl(meth)acrylat oder Hydroxyethyl(meth)acrylat), Styrol und seinen Derivaten (besonders bevorzugt Chlormethylstyrol oder 4-Acetoxystyrol), 2-Vinyl-4,4-dimethylazlacton, substituierten oder unsubstituierten N-Alkyl(meth)acrylamiden und ihren Derivaten und substituierten oder unsubstituierten N,N'-Dialkyl(meth)acrylamiden und ihren Derivaten, aufgebaut sind, da diese wie nachstehend dargestellt auf geeignete Weise synthetisch zugänglich sind. Die Oligomere und/oder Polymere sind miteinander und mit den inneren und äußeren Oberflächen der porösen Matrix über einen oder mehrere Vernetzer, ausgewählt aus der Gruppe, bestehend aus Ethylenglycoldimethacrylat, Trimethylpropantrimethacrylat, Divinylbenzol und N,N'-Methylenbisacrylamid, vernetzt. Bevorzugt weist das mindestens eine Monomer eine Epoxyfunktion auf, da über diese synthetisch einfach zugänglich weitere chromatographisch aktive Zentren wie beispielsweise (Affinitäts)-Liganden eingeführt werden können und/oder über diese die Mikroglobuli einfach an die poröse Matrix gebunden werden können. Bevorzugt sind die Mikroglobuli lediglich aus einem einzigen Monomer aufgebaut.

Bevorzugt sind die unporösen Mikroglobuli in einer Monolage (Monolayer) auf den inneren und äußeren Oberflächen der porösen Matrix gebunden. Sind die unporösen Mikroglobuli hingegen in Multilagen (Multilayers) auf der porösen Matrix gebunden, so besteht die Gefahr, dass sich die Poren der porösen Matrix verstopfen, worunter die Permeabilität des erfindungsgemäßen Chromatographiemediums leiden kann. Erfindungsgemäß beträgt der Pfropfgrad P des Chromatographiemediums von 25% bis 40%. Für den Pfropfgrad P gilt:

$$P = \frac{m_m - m_0}{m_0} * 100\%$$

wobei $m_m$ die Masse des erfindungsgemäßen Chromatographiemediums ist und mo die Masse der porösen Matrix (ohne gebundene Mikroglobuli) ist. Beträgt der Pfropfgrad P mehr als 40%, so kann die Permeabilität des Chromatographiemediums zurückgehen. Beträgt hingegen der Pfropfgrad weniger als 25%, so kann die Oberfläche des Chromatographiemediums im Vergleich zu der porösen (Ausgangs)-Matrix nur in einem geringeren Ausmaß vergrößert werden.

Erfindungsgemäß beträgt der relative Pfropfgrad $P_{rel}$ des Chromatographiemediums höchstens 0,25 $g/cm^3$. Für den relativen Pfropfgrad $P_{rel}$ gilt:

$$P_{rel} = \frac{P * \rho}{Por}$$

wobei P der vorstehend definierte Pfropfgrad des Chromatographiemediums in % ist, $\rho$ die Dichte der porösen Matrix (ohne Mikroglobuli) in $g/cm^3$ ist, und Por die Porosität der porösen Matrix (ohne Mikroglobuli) in % ist. Beträgt der relative Pfropfgrad des Chromatographiemediums wie in den SdT-Dokumenten 1 bis 5 mehr als 0,25 $g/cm^3$, so kann die Permeabilität des Chromatographiemediums zurückgehen.

Gemäß einer Ausführungsform der vorliegenden Erfindung weisen die unporösen Mikroglobuli zusätzliche chromatographisch aktive Zentren bzw. Liganden auf, die an die Mikroglobuli gebunden bzw. auf diesen immobilisiert

sind. Die zusätzlichen chromatographisch aktiven Zentren bzw. Liganden sind in der Lage, mit bestimmten Komponenten von Fluiden in Chromatographieprozessen selektiv Bindungen einzugehen.

Beispielhaft können als chromatographisch aktive Zentren bzw. Liganden Ionenaustauscher, Chelatbildner und Schwermetallchelate, thiophile, hydrophobe Liganden verschiedener Kettenlängen und Konfigurationen, "Reversed Phase"-Systeme, Farbstoffliganden, Affinitätsliganden, Aminosäuren, Coenzyme, Cofaktoren und deren Analoga, Substrate und deren Analoga, endokrine und exokrine Substanzen, wie Hormone und hormonähnlich wirkende Stoffe, Effektoren und deren Analoga, Enzym-Substrate, Enzym-Inhibitoren und deren Analoga, Fettsäuren, Fettsäurederivate, konjugierte Fettsäuren und deren Analoga, Nukleinsäuren, wie DNA, RNA und deren Analoga und Derivate (einzel-, doppel- und/oder mehrsträngig), sowie Peptidnukleinsäuren und deren Derivate, Viren, Virenpartikel, Monomere und deren Analoga und Derivate, Oligo- bis Polymere und deren Analoga und Derivate, hochmolekulare Kohlenhydrate, die linear oder verzweigt, nicht-substituiert oder substituiert sein können, polymere Glycokonjugate, wie Heparin, Amylose, Cellulose, Chitin, Chitosan und deren Mono- und Oligomere und Derivate und Analoga davon, Lignin und dessen Derivate und Analoga, andere biologisch-chemische Liganden, wie Oligo- und Polypeptide, z.B. Proteine und ihre Oligomere, Multimere, Untereinheiten sowie Teile davon, insbesondere Lectine, Antikörper, Fusionsproteine, Haptene, Enzyme und Untereinheiten sowie Teile davon, Strukturproteine, Rezeptoren und Effektoren sowie Teile davon, des Weiteren Xenobiotika, Pharmazeutika und pharmazeutische Wirkstoffe, Alkaloide, Antibiotika, Biomimetika usw. genannt werden. Gemäß einer Ausführungsform der vorliegenden Erfindung sind die zusätzlichen chromatographisch aktiven Zentren bzw. Liganden ausgewählt aus der Gruppe, bestehend aus anionischen und kationischen Gruppen, hydrophoben Gruppen, Affinitätsliganden, Metallchelaten und reaktiven Epoxid-, Aldehyd-, Azlacton-, N-Hydroxysuccinimid- und/oder Carbodiimid-Gruppen. Gemäß einer besonders bevorzugten Ausführungsform der vorliegenden Erfindung sind die zusätzlichen chromatographisch aktiven Zentren bzw. Liganden Protein A oder Protein B.

Ein weiterer Aspekt der vorliegenden Erfindung betrifft ein Verfahren zur Herstellung des erfindungsgemäßen Chromatographiemediums, umfassend
Bereitstellen einer porösen Ausgangsmatrix, wobei die poröse Ausgangsmatrix originär zur radikalischen Polymerisation befähigte Gruppen aufweist, oder die poröse Ausgangsmatrix eine Matrix ist, bei der durch Oberflächenmodifikation funktionelle Gruppen eingeführt wurden;

> Bereitstellen einer Polymerisationslösung, umfassend mindestens ein Monomer, einen bi-, tri- oder multifunktionellen Vernetzer, einen Polymerisationsinitiator und ein Lösungsmittel oder Lösungsmittelgemisch, wobei das mindestens eine Monomer, der bi-, tri- oder multifunktionelle Vernetzer und der Polymerisationsinitiator in dem Lösungsmittel oder Lösungsmittelgemisch vollständig löslich sind; und Initiieren der Polymerisation in der Polymerisationslösung in Anwesenheit der porösen Ausgangsmatrix zur Bildung von unporösen Mikroglobuli, wobei die unporösen Mikroglobuli in dem Lösungsmittel oder Lösungsmittelgemisch unlöslich sind und an die inneren und äußeren Oberflächen der porösen Ausgangsmatrix kovalent gebunden werden;
> wobei der durchschnittliche Radius der Mikroglobuli nicht mehr als 30% des durchschnittlichen Porendurchmessers der porösen Ausgangsmatrix beträgt, wobei der Radius der Poren der porösen Matrix und der Radius der Mikroglobuli über Rasterelektronenmikroskopie bestimmt werden;
> wobei das Gesamtvolumen von Monomer und Vernetzer, bezogen auf das Gesamtvolumen der Polymerisationslösung, maximal 20 Vol.-% beträgt;
> wobei das Gesamtvolumen des Vernetzers, bezogen auf das Gesamtvolumen der Polymerisationslösung, maximal 6 Vol.-% beträgt;
> wobei das mindestens eine Monomer ausgewählt ist aus der Gruppe, bestehend aus Glycidyl(meth)acrylat, substituierten oder unsubstituierten Alkyl(meth)acrylaten und ihren Derivaten, Styrol und seinen Derivaten, 2-Vinyl-4,4-dimethylazlacton, substituierten oder unsubstituierten N-Alkyl(meth)acrylamiden und ihren Derivaten und substituierten oder unsubstituierten N,N'-Dialkyl(meth)acrylamiden und ihren Derivaten;
> wobei der bi-, tri- oder multifunktionelle Vernetzer ausgewählt ist aus der Gruppe, bestehend aus Ethylenglycoldimethacrylat, Trimethylpropantrimethacrylat, Divinylbenzol und N,N'-Methylenbisacrylamid;
> wobei der Polymerisationsinitiator ausgewählt ist aus der Gruppe, bestehend aus 2-Hydroxy-4-(2-hydroxyethoxy)-2-methylpropiophenon, Azobis(isobutyronitril), 4,4'-Azobis(4-cyanovaleriansäure), 1,1'-Azobis(cyclohexancarbonitril), Benzoylperoxid, 2,2-Bis(tert-butylperoxy)butan, 1,1-Bis(tert-butylperoxy)cyclohexan, tert-Butylhydroperoxid, tert-Butylperoxyisopropylcarbonat, Cyclohexanonperoxid und 2,4-Pentandionperoxid;
> wobei das Lösungsmittel oder Lösungsmittelgemisch ausgewählt ist aus der Gruppe, bestehend aus Cyclohexanol/Dodecan-1-ol, Octan-2-on, n-Butylacetat, p-Xylen, Toluol, Ethylacetat, Benzonitril, Cyclohexanon, Dodecan-1-ol, Acetonitril/Ethanol/Wasser, Decan-1-ol und Isopropanol/Decan-1-ol; und wobei die Konzentration des Polymerisationsinitiators in der Polymerisationslösung von 1 bis 3 Gew.-% beträgt.

Sämtliche vorstehende Ausführungen betreffend das erfindungsgemäße Chromatographiemedium gelten auch für das erfindungsgemäße Verfahren zur Herstellung des erfindungsgemäßen Chromatographiemediums. Im ersten

Schritt des erfindungsgemäßen Verfahrens wird eine poröse Ausgangsmatrix bereitgestellt. Es können beispielsweise die vorstehend beim erfindungsgemäßen Chromatographiemedium beschriebenen porösen Matrices verwendet werden. Derartige poröse Ausgangsmatrices sind kommerziell erhältlich oder können durch Verfahren, die dem Fachmann bekannt sind, hergestellt werden. Im zweiten Schritt des erfindungsgemäßen Verfahrens wird eine Polymerisationslösung bereitgestellt, umfassend mindestens ein Monomer, einen bi-, tri- oder multifunktionellen Vernetzer, einen Polymerisationsinitiator und ein Lösungsmittel oder Lösungsmittelgemisch, wobei das mindestens eine Monomer, der bi-, tri- oder multifunktionelle Vernetzer und der Polymerisationsinitiator in dem Lösungsmittel oder Lösungsmittelgemisch vollständig löslich sind.

Es kann sowohl ein einziges Monomer als auch ein Gemisch von zwei oder mehr unterschiedlichen Monomeren verwendet werden. Das mindestens eine Monomer ist vollständig in dem verwendeten Lösungsmittel oder Lösungsmittelgemisch löslich. Erfindungsgemäß ist das mindestens eine Monomer ausgewählt aus der Gruppe, bestehend aus Glycidyl(meth)acrylat, substituierten oder unsubstituierten Alkyl(meth)acrylaten und ihren Derivaten (besonders bevorzugt Methyl(meth)acrylat, Hydroxyethyl(meth)acrylat oder Butyl(meth)acrylat), Styrol und seinen Derivaten (besonders bevorzugt Chlormethylstyrol oder 4-Acetoxystyrol), 2-Vinyl-4,4-dimethylazlacton, substituierten oder unsubstituierten N-Alkyl(meth)acrylamiden und ihren Derivaten und substituierten oder unsubstituierten N,N'-Dialkyl(meth)acrylamiden und ihren Derivaten. Bevorzugt weist das mindestens eine Monomer eine Epoxyfunktion auf, da über diese synthetisch einfach zugänglich weitere chromatographisch aktive Zentren wie beispielsweise (Affinitäts)-Liganden eingeführt werden können und/oder über diese die Mikroglobuli einfach an die poröse Matrix gebunden werden können.

Des Weiteren umfasst die Polymerisationslösung im erfindungsgemäßen Verfahren einen bi-, tri- oder multifunktionellen Vernetzer. Erfindungsgemäß wird unter dem Begriff "Vernetzer" ein Reagens verstanden, das in der Lage ist, die entstehenden Mikroglobuli mit den inneren und äußeren Oberflächen der porösen Ausgangsmatrix zu vernetzen und insbesondere in der Lage ist, die Oligomere bzw. Polymere miteinander zu vernetzen, damit die erfindungsgemäße unporöse mikroglobuläre Struktur entsteht. Der Vernetzer im erfindungsgemäßen Verfahren ist vollständig in dem verwendeten Lösungsmittel oder Lösungsmittelgemisch löslich und ist bi-, tri-oder multifunktionell ist, d.h. er weist zwei, drei oder mehrere funktionelle Gruppen auf, über die das zu bildende dreidimensionale Netzwerk verknüpft wird. Erfindungsgemäß kann sowohl ein einziger als auch ein Gemisch aus zwei oder mehr Vernetzern verwendet werden. Erfindungsgemäß ist der bi-, tri- oder multifunktionelle Vernetzer ausgewählt aus der Gruppe, bestehend aus Ethylenglycoldimethacrylat, Trimethylpropantrimethacrylat, Divinylbenzol und N,N'-Methylenbisacrylamid.

Des Weiteren umfasst die Polymerisationslösung im erfindungsgemäßen Verfahren einen Polymerisationsinitiator. Erfindungsgemäß wird unter dem Begriff "Polymerisationsinitiator" ein Reagens verstanden, das in der Lage ist, beispielsweise durch UV-Bestrahlung oder durch thermische Energiezufuhr, eine radikalische Polymerisation auszulösen. Der Polymerisationsinitiator im erfindungsgemäßen Verfahren ist vollständig in dem verwendeten Lösungsmittel oder Lösungsmittelgemisch löslich. Es kann sowohl ein einziger als auch ein Gemisch aus zwei oder mehr Polymerisationsinitiatoren verwendet werden. Erfindungsgemäß ist der Polymerisationsinitiator ausgewählt aus der Gruppe, bestehend aus 2-Hydroxy-4-(2-hydroxyethoxy)-2-methylpropiophenon, Azobis(isobutyronitril), 4,4'-Azobis(4-cyanovaleriansäure), 1,1'-Azobis(cyclohexancarbonitril), Benzoylperoxid, 2,2-Bis(tert-butylperoxy)butan, 1,1-Bis(tert-butylperoxy)cyclohexan, tert-Butylhydroperoxid, tert-Butylperoxyisopropylcarbonat, Cyclohexanonperoxid und 2,4-Pentandionperoxid.

Im dritten Schritt des erfindungsgemäßen Verfahrens wird eine Polymerisation in der Polymerisationslösung in Anwesenheit der porösen Ausgangsmatrix zur Bildung von unporösen Mikroglobuli initiiert, wobei die unporösen Mikroglobuli in dem Lösungsmittel oder Lösungsmittelgemisch unlöslich sind und an die inneren und äußeren Oberflächen der porösen Ausgangsmatrix kovalent gebunden werden. Die Initiierung der Polymerisation unterliegt keiner besonderen Einschränkung und kann beispielsweise durch UV-Bestrahlung und/oder thermisch erfolgen.

In der Polymerisation werden unporöse Mikroglobuli gebildet, wobei die unporösen Mikroglobuli an die inneren und äußeren Oberflächen der porösen Ausgangsmatrix kovalent (z.B. durch Pfropfpolymerisation) gebunden werden. Die unporösen Mikroglobuli sind in dem verwendeten Lösungsmittel oder Lösungsmittelgemisch unlöslich, d.h. es findet keine Lösung im nachweisbaren Bereich statt.

Das Lösungsmittel oder Lösungsmittelgemisch im zweiten und dritten Schritt des erfindungsgemäßen Verfahrens unterliegt der Einschränkung, dass das mindestens eine Monomer, der bi-, tri- oder multifunktionelle Vernetzer und der Polymerisationsinitiator darin vollständig löslich sind, d.h. eine homogene Polymerisationslösung vorliegt, wohingegen die im dritten Schritt entstehenden Mikroglobuli in dem Lösungsmittel oder Lösungsmittelgemisch vollständig unlöslich sind. Erfindungsgemäß wird daher ein Lösungsmittel oder Lösungsmittelgemisch, ausgewählt aus der Gruppe, bestehend aus Cyclohexanol/Dodecan-1-ol, Octan-2-on, n-Butylacetat, p-Xylen, Toluol, Ethylacetat, Benzonitril, Cyclohexanon, Dodecan-1-ol, Acetonitril/Ethanol/Wasser, Decan-1-ol und Isopropanol/Decan-1-ol, verwendet.

Der durchschnittliche Radius der im dritten Schritt des erfindungsgemäßen Verfahrens entstehenden Mikroglobuli beträgt nicht mehr als 30% des durchschnittlichen Porendurchmessers der porösen Ausgangsmatrix (d.h. der porösen Ausgangsmatrix ohne Mikroglobuli). Dies wird dadurch erreicht, dass die unporösen Mikroglobuli während der Polymerisation aufgrund des schnellen Zerfalls des Polymerisationsinitiators entstehen, der mit dem mindestens einem

Monomer schnell zu Oligo- und Polymeren reagiert. Die daraufhin schnell, vorzugsweise innerhalb von maximal 30 Sekunden, einsetzende makroskopisch beobachtbare Phasenseparation in dem Lösungsmittel oder Lösungsmittelgemisch, in dem sich die wachsenden Polymerketten nicht lösen, führt zur Bildung der unporösen Mikroglobuli. Durch Wechselwirkungen mit der Oberfläche durch das eingesetzte Verhältnis von Monomer und Vernetzer zum Gesamtvolumen der Reaktionslösung und durch die sehr kurze Reaktionszeit bilden sich die unporösen Mikroglobuli nur an den inneren und äußeren Oberflächen der porösen Ausgangsmatrix und bilden kein zusammenhängendes Netzwerk, das die Porenräume der porösen Ausgangsmatrix ausfüllen und somit eine eigene Porosität ausbilden würde. Die Porenstruktur der porösen Ausgangsmatrix bleibt dadurch weitestgehend erhalten, so dass eine Beeinträchtigung der Permeabilität des Chromatographiemediums, wie dies bei einer kompletten Ausfüllung der Poren gemäß der SdT-Dokumente 1 bis 5 der Fall wäre, deutlich reduziert werden kann.

Um die vorstehend beschriebene Ausbildung der Globuli zu erreichen, wird eine Polymerisationslösung verwendet, in welcher das Gesamtvolumen von Monomer und Vernetzer, bezogen auf das Gesamtvolumen der Polymerisationslösung, maximal 20 Vol.-% beträgt. Zudem beträgt das Gesamtvolumen des Vernetzers, bezogen auf das Gesamtvolumen der Polymerisationslösung, maximal 6 Vol.-%. Außerdem beträgt die Konzentration des Polymerisationsinitiators in der Polymerisationslösung von 1 bis 3 Gew.-%. Durch Abstimmung der oben genannten Bedingungen und Bestandteile der Polymerisationslösung kann sichergestellt werden, dass die makroskopisch beobachtbare Phasenseparation nach maximal 30 Sekunden, vorzugsweise nach maximal 20 Sekunden eintritt, so dass an die inneren und äußeren Oberflächen der porösen Ausgangsmatrix kovalent gebundene Mikroglobuli entstehen, deren durchschnittlicher Radius nicht mehr als 30% des durchschnittlichen Porendurchmessers der porösen Ausgangsmatrix beträgt. Unter "makroskopisch beobachtbarer Phasenseparation" wird erfindungsgemäß das Erreichen eines heterogenen, zwei- oder mehrphasigen Zustandes der vorher homogenen, in einer einzigen Phase vorliegenden Polymerisationslösung verstanden. Die Mikroglobulistruktur agglomeriert während der Polymerisation sehr wenig, d.h. die Mikroglobuli vernetzen während ihrer Entstehung nur sehr wenig untereinander, so dass eine Ausfüllung der Poren der porösen Ausgangsmatrix im Gegensatz zu den aus den SdT-Dokumenten 1 bis 5 bekannten Chromatographiemedien weitestgehend verhindert wird.

Durch die unporösen Mikroglobuli auf den inneren und äußeren Oberflächen der porösen Matrix des erfindungsgemäßen Chromatographiemediums wird die spezifische Oberfläche des Chromatographiemediums vorteilhafterweise im Vergleich zu einem Chromatographiemedium, das lediglich aus einer porösen Matrix aufgebaut ist, signifikant erhöht. Dies erlaubt vorteilhafterweise eine höhere Anzahl an chromatographisch aktiven Zentren, so dass das erfindungsgemäße Chromatographiemedium eine erhöhte dynamische Bindungskapazität aufweist. Die Porenstruktur der porösen Matrix bleibt dabei weitestgehend erhalten, so dass die Permeabilität des erfindungsgemäßen Chromatographiemediums im Vergleich zu einem Chromatographiemedium, das lediglich aus einer porösen Matrix aufgebaut ist, vorteilhafterweise wenig beeinträchtigt ist. Demzufolge erlaubt das erfindungsgemäße Chromatographiemedium vorteilhafterweise einen hohen Fluss und weist zudem eine hohe dynamische Bindungskapazität auf. Zudem erlauben die Mikroglobuli vorteilhafterweise eine Optimierung der Porengröße der porösen Matrix im Hinblick auf eine affinitätschromatographische Anwendung, d.h. insbesondere können solche Poren der porösen Matrix optimiert werden, die ohne die unporösen Mikroglobuli eine zu große Porengröße aufweisen würden, so dass ein Zielmolekül im Chromatographieprozess diese Poren konvektiv durchströmen würde, ohne dabei Wechselwirkungen mit der Oberfläche zu zeigen. Durch die unporösen Mikroglobuli entstehen vorteilhafterweise keine Poren, die nur diffusiv erreichbar sind. Dies minimiert vorteilhafterweise eine Abhängigkeit der dynamischen Bindungskapazität von der Verweilzeit. Zudem wird dadurch die Zugänglichkeit zu den chromatographisch aktiven Zentren in einem dynamischen Trennprozess erhöht, so dass insbesondere der Verbrauch an nachträglich in das Chromatographiemedium eingeführten chromatographisch aktiven Zentren wie beispielsweise (Affinitäts)-Liganden minimiert werden kann, da die Liganden während ihrer Immobilisierung (Einführung) nicht in für das Zielmolekül der Chromatographie unzugängliche Poren immobilisiert werden. Das erfindungsgemäße Chromatographiemedium eignet sich daher hervorragend zur Anwendung in der Affinitätschromatographie, insbesondere für die Protein A oder B Affinitätschromatographie.

Die vorliegende Erfindung wird anhand der folgenden, nicht einschränkenden Beispiele näher erläutert.

**Beispiele**

<u>Poröse Ausgangsmatrices:</u>

Die in dem erfindungsgemäßen Verfahren als Ausgangsmatrices verwendeten porösen Matrices mit einer Porengröße von 0,01 bis 20 $\mu$m, vorzugsweise 0,1 bis 10 $\mu$m und mehr bevorzugt von 0,2 bis 4 $\mu$m wurden durch übliche, dem Fachmann bekannte Herstellungsverfahren hergestellt. Zur Bestimmung der Porengröße wurde ein "Capillary Flow Porometry Test" durchgeführt. Details für die Bestimmung sind der entsprechenden Bedienungsanleitung (Capillary Flow Porometer 6.0, CAPWIN Software System, Fa. Porous Materials Inc.) zu entnehmen.

Beispiel 1: Vorbehandlung der Oberflächen

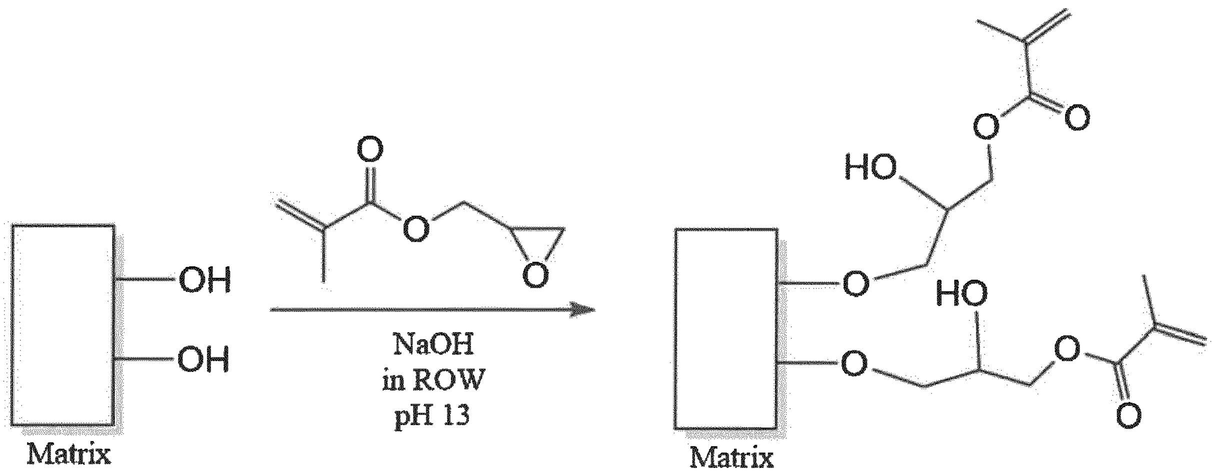


Vorbehandlung des Cellulose-Ausgangsmaterials zur Erzeugung von ungesättigten Funktionalitäten auf der Oberfläche der porösen Ausgangsmatrix

In einer typischen Umsetzung wurde das Ausgangsmaterial, regenerierte stabilisierte Cellulose, in einer 20 Gew.-%-Lösung aus Glycidylmethacrylat in Wasser, die mit Natriumhydroxid auf pH 13 eingestellt wurde, über Nacht imprägniert und danach 15 Minuten mit Umkehrosmosewasser ("RO-Wasser"; reversed osmosis water) gespült. Anschließend wurden die behandelten Membranen bei 80 °C für 30 Minuten getrocknet.

Beispiel 2: Erzeugung des Pfropfpolymers auf der behandelten Oberfläche

Für eine typische Polymerisationslösung wurden beispielsweise 1,7 mL (1,82 g; 12,79 mmol; 0,10 Äq.) Glycidylmethacrylat (Monomer), 0,16 g (0,71 mmol; $7\cdot10^{-3}$ Äq.) 2-Hydroxy-4-(2-hydroxyethoxy)-2-methylpropiophenon (Polymerisationsinitiator), 0,33 mL (0,347 g; 1,75 mmol; 0,013 Äq.) Ethylenglycoldimethacrylat (Vernetzer), 0,34 mL (0,28 g; 1,78 mmol; 0,014 Äq.) 1-Decanol und 13,36 mL (12,85 g; 128,32 mmol; 1,00 Äq.) Cyclohexanol (Lösungsmittelgemisch) eingewogen bzw. abgemessen und bis zum vollständigen Lösen 30 Minuten im Ultraschallbad behandelt. Anschließend wurden die mit Polymerisationslösung benetzten Proben unter inerten Bedingungen ($N_2$-Atmosphäre) mit UV-Licht (Ultraviolettes Licht; 254 nm, 40 W) bestrahlt (5 Minuten). Für die hierbei ablaufende UV-Initiierte, konventionelle, radikalische Polymerisation wurde ein UV-Crosslinker BLX-E254 BIO-Link des Herstellers *LLG* verwendet. Die auspolymerisierten Membranen wurden abschließend 10 Minuten unter laufendem RO-Wasser gespült und anschließend für 20 Minuten in Aceton geschüttelt und an Luft getrocknet.

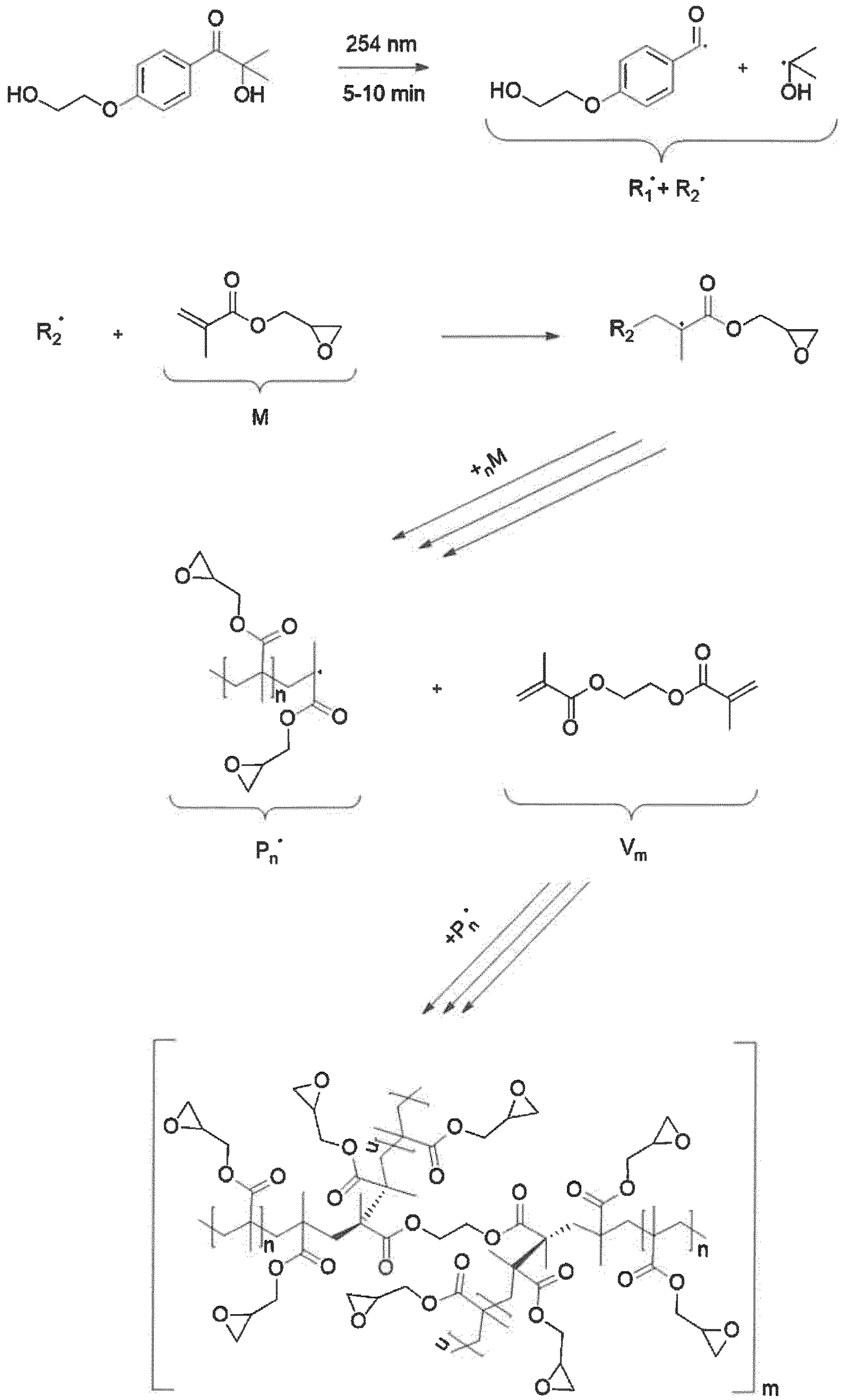


Schematische Darstellung des Polymerisationsmechanismus.

Folgende konkrete Ausführungsbeispiele mit Angabe der verwendeten Chemikalie und der jeweils verwendeten Menge in Vol.-% und Gew.-% wurden durchgeführt:

| | Initiator [Gew.-%] | Vernetzter **[Vol.-%]** | Monomer **[Vol.-%]** | Lösungsmittel 1 **[Vol.-%]** | Lösungsmittel 2 **[Vol.-%]** |
|---|---|---|---|---|---|
| Erfindungsgemäße Modifizierung 1 | 2-Hydroxy-4-(2-hydroxy-ethoxy)-2-methyl propiophenon/**1** | Ethylenglycold i-methacrylat/**4** | Glycidylmethacrylat/ **16** | 1-Decanol/**9** | Cyclohexanol/**70** |
| Erfindungsgemäße Modifizierung 2 | 2-Hydroxy-4-(2-hydroxy-ethoxy)-2-methylpropiophenon/**1** | Ethylenglycoldimethacrylat/ **6** | Glycidylmethacrylat/ **14** | 1-Decanol/**3** | Cyclohexanol/**76** |
| Erfindungsgemäße Modifizierung 3 | 2-Hydroxy-4-(2-hydroxy-ethoxy)-2-methyl propiophenon/**1** | Ethylenglycold i-methacrylat/**5** | Glycidylmethacrylat/ **15** | 1-Decanol/**10** | Cyclohexanol/**69** |
| Erfindungsgemäße Modifizierung 4 | 2-Hydroxy-4-(2-hydroxy-ethoxy)-2-methylpropiophenon/**1** | Ethylenglycoldimethacrylat/ **2** | Glycidylmethacrylat/ **18** | 1-Decanol/15 | Cyclohexanol/**64** |

Figur 1 zeigt REM-Aufnahmen des erfindungsgemäßen Chromatographiemediums durch die erfindungsgemäße Modifizierung 2 (oben/mittig) und der porösen Ausgangsmatrix vor der Modifizierung (unten). Figur 1 zeigt deutlich, dass die Mikroglobuli lediglich auf die Oberflächen der Ausgangsmatrix gepfropft sind und die eigentliche Porenstruktur der Ausgangsmatrix nahezu erhalten bleibt. Zudem ist deutlich ersichtlich, dass die Mikroglobuli selbst keine Porosität aufweisen.

Figur 2 hingegen zeigt REM-Aufnahmen von Chromatographiemedien, die bei gleicher Ausgangsmatrix gemäß den Modifizierungsprotokollen der SdT-Dokumente 2 (links oben), 5 (rechts oben), 1 (links unten) und 4 (rechts unten) modifiziert wurden. Unter diesen Modifizierungsbedingungen stellt sich keine differenzierte Mikroglobulistruktur ein und es ist gut zu erkennen, dass die Poren der porösen Ausgangsmatrices komplett gefüllt werden und die globulären Strukturen, falls überhaupt vorhanden, sehr stark agglomeriert sind, so dass die effektive Oberfläche stark herabgesetzt ist und eine vom Monomer erzeugte zusätzliche Porosität in den Poren der Ausgangsmatrices entsteht.

Beispiel 3: Permeabilitätsbestimmung der Chromatographiemedien

Aus den Chromatographiemedien gemäß Beispiel 2 wurden mit einem Rundstanzeisen 47 mm Ronden ausgestanzt. Die erhaltenen 47 mm Stanzlinge wurden mit Umkehrosmosewasser benetzt und für 5 Minuten unter fließendem Umkehrosmosewasser gespült. Der jeweilige Stanzling wurde in ein Aufgussfiltrationsgehäuse Sartorius Typ 16249 eingebaut.

Die Messung erfolgte jeweils bei 20-25°C und 0,10 bar Überdruck. Es wurde die Zeit gemessen, die 100,0 g Medium benötigen, um durch das Chromatographiemedium zu strömen. Die Einheit des so ermittelten Durchflusses wird angegeben mit ml/cm$^2$ $\times$ min x bar. Als Medien werden sowohl Umkehrosmosewasser als auch phosphatgepufferte Salzlösung mit einem pH-Wert von 7,5 verwendet. Die Stanzlinge konnten nach der Bestimmung des Durchflusses zu weiteren Untersuchungen verwendet werden.

Figur 3 zeigt, dass durch die erfindungsgemäße Modifizierung 2 ("Erfindungsg. Mod. 2") der porösen Ausgangsmatrix durch Bildung von Mikroglobuli auf den inneren und äußeren Oberflächen der Ausgangsmatrix die Permeabilität mindestens 40% der Permeabilität der Ausgangsmatrix ohne Modifizierung beträgt. Findet hingegen eine Modifizierung der Ausgangsmatrix gemäß den Modifizierungsprotokollen der SdT-Dokumente 1 bis 5 statt ("Mod. SdT 1 bis 5"), so beträgt die Permeabilität des modifizierten Chromatographiemediums maximal 26% der Permeabilität der Ausgangsmatrix ohne Modifizierung, meistens sogar deutlich unter 20% der Ausgangspermeabilität.

Beispiel 4: Immobilisierung von Proteinen

Die Immobilisierung von Proteinen (Liganden) erfolgte durch Umsetzen der Epoxidfunktionalitäten der Mikroglobuli der erfindungsgemäßen Chromatographiemedien zu Aldehydfunktionalitäten. An diesen konnte das Protein (der Ligand) direkt immobilisiert werden. Hierfür wurde das erfindungsgemäß modifizierte Chromatographiemedium 30 min in eine 3%ige (Gew.-%) Natriummetaperiodatlösung in RO-Wasser gelegt und nachfolgend 10 Minuten in fließendem RO-Wasser gespült und aus Aceton an Luft getrocknet. Anschließend wurden die so präparierten Chromatographiemedien 2 h in eine 2,5%ige (Gew.-%) Natriumcyanoborhydridlösung in Kaliumhydrogenphosphatpuffer (pH 8) gelegt, zu der 4,958 $\mu$L/cm$^2$ rekombinante Protein-Lösung (1 mL $\hat{=}$ 50,4 mg Protein) gegeben wurden, um eine Konzentration von 7,2 mg/mL zu erreichen. Anschließend wurden die Chromatographiemedien erneut mit RO-Wasser gespült und für 10 min in eine 0,01%ige (w%) Natriumborhydridlösung in RO-Wasser gelegt. Anschließend wurden die Chromatographiemedien mit 1 $\times$ PBS-Puffer (phosphatgepufferte Kochsalzlösung, "phosphate buffered saline solution") gewaschen und nachfolgend in einer 20%igen Ethanol/PBS Lösung gelagert.

Beispiel 5: Bestimmung der Bindungskapazität für Immunglobulin G:

Für die Bestimmung der Bindung von Immunglobulin G (IgG) wurde eine entsprechende Proteinlösung (1 mg/mL) in PBS-Puffer (pH =7,5) verwendet. 3 Lagen Membran, erfindungsgemäß modifiziert nach Beispiel 2 und funktionalisiert mit Protein A nach Beispiel 4, wurden in einen Membranhalter eingespannt. Der Membranstapel wies eine Membranfläche von 15 cm$^2$, eine Anströmfläche von 5 cm$^2$ und eine Betthöhe (Dicke des Membranstapels) von 500 $\mu$m in dem Membranhalter auf. Die Membranen in dem Membranhalter wurden mit 1 $\times$ PBS-Puffer (pH =7,5) geflutet, um die Luft zu verdrängen und dann an eine FPLC-Anlage Äkta Explorer 100 der Firma General Electric Health Care angeschlossen.

Danach wurden die Membranen bzw. der Membranstapel mit einem vier Schritte umfassenden Testprogramm hinsichtlich der IgG-Bindung untersucht. Die vier Schritte des Testprogramms sind nachfolgend angegeben:

1. Äquilibrieren der Membran mit 25 ml 1xPBS-Puffer (pH =7,5),
2. Beladen der Membran mit 50 ml IgG-Lösung,

3. Waschen mit 20 ml 1xPBS-Puffer (pH =7,5), und
4. Eluieren mit 0,1% Glycin Lösung (pH =3,5).

Alle Schritte wurden bei einer Flussgeschwindigkeit von 5 Membranvolumen/min durchgeführt. Bei allen Schritten wurde die Absorption bei 280 nm im Detektor hinter der Membraneinheit gemessen.

Figur 4 zeigt die Durchbruchskurven der erfindungsgemäß hergestellten Chromatographiemedien nach erfindungsgemäßer Modifizierung 1, aufgetragen als prozentualer Durchbruch gegen die dynamische Bindungskapazität DBC. Als Referenz ist die Durchbruchskurve eines kommerziell erhältlichen Protein A Membranadsorbers (Sartobind A) aufgetragen. Figur 4 zeigt deutlich, dass durch die erfindungsgemäße Modifizierung, d.h. durch die Ausbildung von Mikroglobuli, die spezifische Oberfläche des erfindungsgemäßen Chromatographiemediums signifikant erhöht ist, was vorteilhafterweise zu einer deutlich höheren dynamischen Bindungskapazität führt.

Beispiel 6: Bestimmung der spezifischen Oberfläche der modifizierten Membranen

gemäß Beispiel 2:

Die durchgeführten Oberflächenbestimmungen wurden mithilfe eines Gemini V der Firma *micromeritics* durchgeführt. Die zu messenden Proben wurden zuvor abgewogen und in die Probenkammer gegeben. Diese wurde daraufhin bei 70 °C und 2 mbar (Unterdruck erzeugt durch eine VacPrep 061 der Firma *micromeritics)* für 2 h ausgeheizt. Im Anschluss wurde die Probenkammer mit Stickstoff gespült und erneut bei 70 °C und 2 mbar 1 h ausgeheizt. Zur eigentlichen Messung wurde Stickstoff über das zu untersuchende Material geleitet. Aufgrund von Kühlung durch flüssigen Stickstoff (-196 °C) konnte mit einem Normaldruckmessgerät unterhalb des Sättigungsdampfdruckes des Stickstoffs die adsorbierte Menge bestimmt werden (Adsorption). Anschließende Verringerung des Drucks innerhalb der Apparatur löste einen Teil der adsorbierten Gasmenge von der Oberfläche (Desorption). Dadurch konnte eine Adsorptions-Desorptions-Isotherme ermittelt werden. Im Relativdruckbereich von 0,05 mbar bis 0,3 mbar ist die dabei gemessene Menge an adsorbiertem oder freiwerdendem Gas proportional zur Oberfläche. Die Resultate wurden direkt in $m^2/g$ ausgegeben.

Figur 5 zeigt eine Auftragung der spezifischen Oberflächen nach erfindungsgemäßer Modifizierung und vor Modifizierung (d.h. der porösen Ausgangsmatrices ohne Mikroglobuli). Unabhängig von der spezifischen Oberfläche der Ausgangsmatrix ist die spezifische Oberfläche des erfindungsgemäßen Chromatographiemediums durch Ausbildung der Mikroglobulistruktur immer größer als vor der Modifizierung. Wie aus Figur 6 ersichtlich, führt bei sonst gleichen Bedingungen die erfindungsgemäße Modifizierung vorteilhafterweise zu erhöhten dynamischen Bindungskapazitäten von IgG auf Protein A Membranadsorbern (Immobilisierung nach Beispiel 4).

Beispiel 7: Bestimmung der Porengrößen der erfindungsgemäßen Chromatographiemedien und Bestimmung der Porositäten mittels Inverser Größenausschlusschromatoqraphie (ISEC)

Zur Bestimmung der Porengröße wurde ein "Capillary Flow Porometry Test" durchgeführt. Details sind der entsprechenden Bedienungsanleitung (Capillary Flow Porometer 6.0, CAPWIN Software System, Fa. Porous Materials Inc.) zu entnehmen.

Alle Experimente zur Bestimmung der Porositäten wurden an einem Standard Agilent LC System (Agilent Technologies), bestehend aus einer quaternären Pumpe mit Entgaser, einem temperiertem Autosampler, einem Dioden-Array-Detektor (DAD) und einem Brechungsindex (RI) Detektor, durchgeführt. Acetontracer-Signale wurden mittels des DAD bei einer Wellenlänge von 280 nm aufgenommen. Pullulantracer-Signale wurden mittels RI Detektor verfolgt. Für alle aufgenommenen Signale wurde das erste und zweite statistische Moment gemessen und wie von Haynes und Sarma et al. (AIChE Journal, Volume 19, No. 5, p. 1043-1046) vorgeschlagen berechnet. Die so erhaltenen Werte wurden zudem korrigiert, um den Einfluss des HPLC Systems zu berücksichtigen:

$$\mu_{p,obs} = \frac{\int_0^\infty C_i(L,t) \cdot t \cdot dt}{\int_0^\infty C_i(L,t) \cdot dt}$$

$$\sigma_{p,obs}^2 = \frac{\int_0^\infty C_i(L,t) \cdot \left(t - \mu_{p,obs}\right)^2 \cdot dt}{\int_0^\infty C_i(L,t) \cdot dt}$$

$$\mu_p = \mu_{p,obs} - \mu_{HPLC}$$

$$\sigma_p^2 = \sigma_{p,obs}^2 - \sigma_{HPLC}^2$$

wobei $\mu_p$ und $\sigma_p^2$ für das erste und zweite statistische Moment des Tracersignals stehen, $\mu_{p,obs}$ und $\sigma_{p,obs}^2$ sich auf das ganze System beziehen, während $\mu_{HPLC}$ und $\sigma_{HPLC}^2$ nur den Einfluss des HPLC Systems beschreiben. $C_i(L,t)$ beschreibt die Konzentration von Tracersubstanz *i* am Detektor zum Zeitpunkt *t*.

Um die Porosität des jeweiligen chromatographischen Mediums in Abhängigkeit der Tracermolekülgröße zu bestimmen, wurden Pullulanmoleküle mit unterschiedlichen molaren Massen als Tracersubstanzen verwendet. Ihr hydrodynamischer Radius wurde anhand der molaren Masse berechnet, wie von Segre et al. (Biomacromolecules 2003, 4, 1843-1847) vorgeschlagen. Der zugängliche Volumenanteil im chromatographischen Bett in Abhängigkeit der Molekülgröße wurde wie folgt berechnet:

$$\varepsilon = \frac{V}{F/\mu_p}$$

Wobei $\varepsilon$ für den zugänglichen Volumenanteil (Porosität) steht, V für das Säulenvolumen und F für die volumetrische Flussrate.

Figur 7 zeigt die Porengrößenverteilung von erfindungsgemäßen Chromatographiemedien (nach erfindungsgemäßer Modifizierung 1) gegenüber den verwendeten porösen Ausgangsmatrices (oben) und eine Auftragung der Porosität gegen den hydrodynamischen Durchmesser des Tracermoleküls, ermittelt über ISEC, für Ausgangsmatrices und eine erfindungsgemäß modifiziertes Chromatographiemedium (unten). Wie aus Figur 7 ersichtlich kann erfindungsgemäß die Porengröße der Ausgangsmatrix je nach Anwendung angepasst werden, und nach erfindungsgemäßer Modifizierung weisen die Chromatographiemedien in jedem Fall eine kleinere Porengröße auf als die Ausgangsmatrices. Diese kommt lediglich durch eine Verkleinerung der bereits vorhandenen Poren der Ausgangsmatrices durch Aufpfropfung der unporösen Mikroglobuli zustande. Figur 7 zeigt zudem über Vergleichsmessungen verschiedener Ausgangsmatrices mit gleicher Modifizierung, dass eine Porosität der Mikroglobuli ausgeschlossen werden kann. Wären die Mikroglobuli nämlich porös oder würde sich eine Verstopfung der Poren der Ausgangsmatrix mit einem vernetzten makroporösen Gel zeigen, müsste sich trotz der verschiedenen Ausgangsmaterialien bei gleicher Modifizierung eine Überschneidung der Porenverteilung zeigen, was jedoch nicht der Fall ist.

Beispiel 8: Bestimmung des Quell- und Pfropfgrades erfindungsgemäßer Chromatographiemedien

Der Quellgrad beschreibt den relativen Volumenzuwachs der Polymermembran beim Quellen in Lösemitteln. Aus praktischen Gründen wurde er für Protein A Affinitätsadsorber in PBS-Puffer bestimmt. Hierfür wurden zum einen die Volumina der Chromatographiemedien in trockenem Zustand ($V_0$) und zum anderen in nassem Zustand ($V_Q$) bestimmt. Um Messungenauigkeiten durch den Wassergehalt möglichst auszuschließen, wurde die Messung des trockenen Volumens nach Trocknung im Trockenschrank bei 80 °C (30 min) vorgenommen. Der Quellgrad Q ist über folgende Formel bestimmt worden:

$$Q\,[\%] = \frac{V_Q - V_0}{V_0} \cdot 100\,\%$$

Die nachfolgende Tabelle zeigt die Ergebnisse aus Quellungs- und Durchflussmessungen mit Wasser und Aceton gemäß Beispiel 3 und Beispiel 8, gemessen an einem Chromatographiemedium nach erfindungsgemäßer Modifizierung 1 und an der porösen Ausgangsmatrix (Cellulosemembran) ohne Modifizierung:

| | Cellulose-membran | Cellulosemembran erfindungsgemäß modifiziert |
|---|---|---|
| Quellung $H_2O$ [%] | 22,15±1,1 % | 5,97±1,1 % |
| Quellung Aceton [%] | 4,52±1,1 % | 6,18±1,1 % |

(fortgesetzt)

| | Cellulose-membran | Cellulosemembran erfindungsgemäß modifiziert |
|---|---|---|
| Permeabilität $H_2O$ [mD] | / | 2,65±0,08 % |
| Permeabilität Aceton [mD] | / | 2,70±0,12 % |

Eine Quellung in Wasser als hydrophilem Lösungsmittel, die die Ausgangsmaterialien aufgrund ihrer hydrophilen Struktur zeigen, wird durch die aufgepfropften Mikroglobuli weitestgehend unterbunden, während in Aceton als aprotischem Lösungsmittel nur eine sehr geringe und hauptsächlich durch die Ausgangsmatrix verursachte Quellung stattfindet. Auch die Permeabilität zeigt in beiden Lösungsmitteln keinen signifikanten Unterschied. Dies schließt zusätzlich eine Quellung aus. Per Definition können die aufgepfropften Mikroglobuli somit nicht als polymeres Gel/Hydrogel angesehen werden, im Gegensatz zu den Polymeren aus den SdT-Dokumenten 1 bis 5.

Der Pfropfgrad beschreibt den relativen Massenzuwachs der Ausgangsmatrix nach der Modifizierung. Hierfür wurden zum einen die Massen der Ausgangsmatrix vor der Modifizierung (mo) und zum anderen nach der Modifizierung ($m_m$) bestimmt. Um Messungenauigkeiten durch den Wassergehalt möglichst auszuschließen, wurde die Messung der Masse nach Trocknung im Trockenschrank bei 80 °C (30 min) vorgenommen. Der Pfropfgrad P ist über folgende Formel bestimmt worden:

$$P\,[\%] = \frac{m_m - m_0}{m_0} \cdot 100\,\%$$

Figur 8 zeigt eine Auftragung der Permeabilität (Durchfluss), des Pfropfgrades P und der dynamischen Bindungskapazität eines Protein A Membranadsorbers für IgG gegen die Polymerisationszeit der Modifizierung einer Ausgangsmatrix. Der Pfropfgrad kann dabei sowohl über die Wahl der Komponenten bei der Modifizierung als auch über die Reaktionsdauer eingestellt werden. Aus Figur 8 wird ersichtlich, dass die erfindungsgemäße Zusammensetzung sehr schnelle Reaktionszeiten ermöglicht, die zu den erfindungsgemäßen Strukturmerkmalen führen. Mit steigender Polymerisationszeit erhöht sich der Pfropfgrad im selben Maße, wie die Permeabilität für Wasser sowie die dynamische Bindungskapazität für IgG abnehmen. Ein hoher Pfropfgrad zeigt im erfindungsgemäßen Fall ein Maximum, welches, wenn es überschritten wird, zu einer schlechteren Leistung des Chromatographiemediums führt. Insbesondere die in den SdT-Dokumenten offenbarte lange Polymerisationszeit von 120 Minuten hat ausschließlich negative Effekte auf die Leistung des Chromatographiemediums (siehe auch Beispiel 9).

Wie vorstehend ausgeführt, kann der Pfropfgrad auch als relativer Pfropfgrad über folgenden Gleichung beschriebene werden:

$$P_{rel} = \frac{P * \rho}{Por}$$

wobei P der vorstehend definierte Pfropfgrad des Chromatographiemediums in % ist, ρ die Dichte der porösen Matrix ohne Mikroglobuli in g/cm$^3$ ist, und Por die Porosität der porösen Matrix ohne Mikroglobuli in % ist.

Figur 9 zeigt eine Auftragung der Permeabilität von erfindungsgemäßen Chromatographiemedien (Rauten) und Chromatographiemedien aus den genannten SdT-Dokumenten 1 bis 5 (Quadrate) gegen deren jeweiligen relativen Pfropfgrad. Der relative Pfropfgrad der Chromatographiemedien in den genannten SdT-Dokumenten 1 bis 5 beträgt mehr als 0,25 g/cm$^3$, was zu einem deutlichen Rückgang der Permeabilität der Chromatographiemedien führt. Im Gegensatz dazu zeigen die erfindungsgemäßen Chromatographiemedien aufgrund ihres geringeren Pfropfgrades vorteilhafterweise eine höhere Permeabilität.

Zudem zeigt die Korrelation zwischen Permeabilität und relativem Pfropfgrad sehr deutlich, dass die unporösen Mikroglobuli die Poren der Ausgangsmatrix des erfindungsgemäßen Chromatographiemediums lediglich verengen, da eine lineare Korrelation gefunden wird. Zudem kann beispielhaft anhand zweiter Messpunkte dieser linearen Korrelation ein Verhalten nach *Hagen-Poiseuille* zwischen der ermittelten Porengröße und dem Durchfluss (Permeabilität) gezeigt werden:

Die *Hagen-Poiseuille*-Gleichung beschreibt im Allgemeinen die Änderung des Volumenstroms bei laminaren stationären Strömungen durch ein Rohr bei Änderung des Radius und der Länge des Rohres, wenn das Fluid und deren Eigenschaften unverändert bleiben. Da erfindungsgemäß die Länge der Membran unverändert bleibt und sich lediglich der Durchmesser der Poren durch die Modifizierung mit Mikroglobuli verengt, kann hier davon ausgegangen werden, dass

das postulierte Verhalten und die damit zusammenhängende erfindungsgemäß postulierte strukturelle Beschaffenheit des erfindungsgemäßen Chromatographiemediums (unporöse Mikroglobuli, die die Poren der Ausgangsmatrix lediglich verengen und nicht verstopfen) zutrifft.

Tabelle: Experimentelle Porengrößen und Flussbestimmung im Vergleich mit der errechneten Flussbestimmung nach Hagen-Poiseuille.

| | Ausgangsmatrix | Chromatographiemedium nach erfindungsgemäßer Modifizierung | | Medium nach Modifizierung gemäß SdT-Dokument | |
|---|---|---|---|---|---|
| | | 1 | 2 | 2 | 4 |
| Porengröße [$\mu$m] | 0,41 | 0,27 | 0,35 | 0,186 | 0,31 |
| Experimenteller Durchfluss $H_2O$ [ml/cm$^2$*bar*min] | 36 | 7 | 19 | 6 | 7 |
| Faktor Porengrößenverkleinerung | / | 0,658 | 0,853 | 0,53 | 0,75 |
| Errechneter Durchfluss $H_2O$ nach Modifizierung nach Hagen-Poiseuille [ml/cm$^2$*bar*min] | 36 | 6,77 | 19,11 | 2,98 | 11,76 |

$$\dot{V} = \frac{\pi \cdot r^4 \cdot \Delta p}{8 \cdot \eta \cdot l}$$

Wie die vorstehende Tabelle zeigt, stimmen beim erfindungsgemäßen Chromatographiemedium berechneter und experimenteller Durchfluss sehr gut überein, wohingegen dies bei den Chromatographiemedien aus den SdT-Dokumenten nicht zu beobachten ist. Dies lässt darauf schließen, dass die Poren der Ausgangsmatrix bei der erfindungsgemäßen Modifizierung mit steigendem Pfropfgrad immer weiter verengt werden, wohingegen die makroporösen Gele in den Chromatographiemedien aus den SdT-Dokumenten die Poren komplett füllen, und der Fluss somit von der Porosität der makroporösen Gele bestimmt wird, da hier kein lineares Verhalten beobachtet werden kann. Zudem ist der Fluss (die Permeabilität) der Chromatographiemedien in den SdT-Dokumenten kleiner als jener der erfindungsgemäßen Chromatographiemedien.

Beispiel 9: Bestimmung der immobilisierten chromatographisch aktiven Zentren (Liganden) an Affinitätsadsorbern

Für die Bestimmung eines immobilisierten Proteins als chromatographisch aktives Zentrum (Ligand) pro Membranfläche wurde ein BCA *(bicinchoninic acid)* Test verwendet. Hierfür wurden Stanzlinge (d = 13 mm) aus erfindungsgemäßen Chromatographiemedien ausgestanzt, diese in Petrischalen (3 mL) vorgelegt und mit dem BCA-Reagenz (2 mL) benetzt. Das BCA-Reagenz bestand aus dem BCA Protein Assay Reagent A (Pierce Prod. Nr. 23221) und dem BCA Protein Assay Reagent B (Pierce Prod. Nr. 23224) im Verhältnis 50:1. Anschließend wurden die so präparierten Proben 60 Minuten auf einer Schüttelplattform (80 UpM) bewegt.

Die aus der Oxidation des BCA-Reagenzes folgende Verfärbung wurde als Extinktion (bei 562 nm) gegenüber einer Referenzlösung des BCA-Reagenzes ohne Membranprobe gemessen. Die photometrischen Messungen erfolgten an einem UV/Vis-Spektralphotometer Specord 200 PLUS von *Analytik Jena.* Die Extinktion konnte mittels der Kalibriergerade aus Figur 10, aus der nachstehende Gleichung hervorging, in Masse Protein [$\mu$g] umgerechnet werden.

Dieser Wert wurde im Anschluss auf das Volumen des Stanzlings bezogen und in $\mu$g Protein/mL Membranvolumen ($V_M$) angegeben.

$$\text{Protein A:}\quad A\,[\mathrm{Au}] = 0{,}00578 \cdot \mathrm{Imm.\ Menge\ Protein}\,[\mu g]$$

Figuren 11 bis 13 zeigen Auftragungen der dynamischen Ligandenausnutzung (Figur 11) bzw. der statischen Ligandenausnutzung (Figur 12) bzw. der Menge an immobilisiertem Ligand (Figur 13) gegen die jeweilige spezifische Oberfläche von erfindungsgemäßen Chromatographiemedien.

Wie in den Figuren 11 bis 13 zu erkennen ist, sorgt die durch die Mikroglobuli erzeugte größere spezifische Oberfläche der erfindungsgemäßen Chromatographiemedien für eine Mehrzahl an gebundenen Liganden und überraschenderweise für eine bessere Ligandenausnutzung.

Wenn die Leistung "*Per*" von Chromatographiemedien wie folgt definiert wird, ergibt sich eine deutliche Steigerung der Leistung gegenüber kommerziellen Affinitätsadsorbern und jenen aus den genannten SdT-Dokumenten 1 bis 5, wie die nachstehende Tabelle zeigt.

$$Per\ [\frac{mg \cdot mD}{mL}] = dyn.Bindungskapazität_{10\%}\ [\frac{mg}{mL}] \cdot Permeabilität_{H_2O}\ [mD]$$

Tabelle: Zusammenfassung des Pfropfgrades, der Permeabilität von $H_2O$, der dynamischen Bindungskapazität bei 10% Durchbruch ($DBC_{10\%}$) und der sich daraus ergebenden Leistung der Chromatographiemedien.

| Probe gemessen bei 5 CV/min | Pfropfgrad [%] | $DBC_{10\%}$ [mg/mL] | $Permeabilität_{H2O}$ [mD] | *Per* [mg*mD/ mL] | Ligandenausnutzung $dynamisch_{10\%}$/ statisch |
|---|---|---|---|---|---|
| Erfindungsgemäße Modifizierung 1 | 40 | 21 | 2,7 | 56,70 | 1,5/2,0 |
| Erfindungsgemäße Modifizierung 2 | 25 | 17 | 5,7 | 96,90 | 1,3/1,8 |
| Modifizierung nach SdT-Dokument 1 | 112 | 10 | 0,64 | 6,40 | 0,8/1,4 |
| Modifizierung nach SdT-Dokument 2 | 65 | 9 | 1,92 | 17,27 | 0,7/1,4 |
| Modifizierung nach SdT-Dokument 3 | 50 | 2 | 0,32 | 0,64 | 0,2/0,5 |
| Modifizierung nach SdT-Dokument 4 | 70 | 7 | 2,24 | 15,68 | 0,8/1,4 |
| Modifizierung nach SdT-Dokument Nr.5 | 170 | 2 | 0,009 | 0,018 | 0,2/1 |
| Kommerziell erhältlicher Protein A Membranadsor ber (Sartobind A - Sartorius Stedim Biotech GmbH) | / | 6 | 8,00 | 44,00 | 1/1,5 |

("5 CV/min" entspricht einem Durchsatz von 5 Säulenvolumina pro Minute)

Die Figuren zeigen:

Figur 1: REM-Aufnahmen des erfindungsgemäßen Chromatographiemediums nach erfindungsgemäßer Modifizierung 2 (oben/mittig) und der porösen Ausgangsmatrix vor der Modifizierung (unten).

Figur 2: REM-Aufnahmen von Chromatographiemedien gemäß den Modifizierungsprotokollen der SdT-Dokumente 2 (links oben), 5 (rechts oben), 1 (links unten) und 4 (rechts unten).

Figur 3: Reduzierung der Permeabilität gemäß Beispiel 3.

Figur 4: Durchbruchskurven von erfindungsgemäßen Chromatographiemedien und von Sartobind A, aufgetragen als prozentualer Durchbruch gegen die dynamische Bindungskapazität DBC.

Figur 5: Auftragung der spezifischen Oberflächen nach erfindungsgemäßer Modifizierung und vor Modifizierung.

Figur 6: Auftragung der dynamischen Kapazität von erfindungsgemäßen Chromatographiemedien gegen deren spezifische Oberfläche.

Figur 7: Porengrößenverteilung erfindungsgemäßer Chromatographiemedien und unmodifizierter Ausgangsmatrices (oben); Auftragung Porosität erfindungsgemäßer Chromatographiemedien gegen den hydrodynamischen Durchmesser von Tracermolekülen (unten).

Figur 8: Auftragung der Permeabilität (des Durchflusses), des Pfropfgrads und der dynamischen Bindungskapazität gegen die Polymerisationszeit der Modifizierung.

Figur 9: Auftragung der Permeabilität gegen den relativen Pfropfgrad.

Figur 10: Kalibrierungsgerade für Beispiel 9.

Figur 11: Auftragung der dynamischen Ligandenausnutzung gegen die jeweilige spezifische Oberfläche von erfindungsgemäßen Chromatographiemedien.

Figur 12: Auftragung der statischen Ligandenausnutzung gegen die jeweilige spezifische Oberfläche von erfindungsgemäßen Chromatographiemedien.

Figur 13: Auftragung der Menge an immobilisiertem Ligand gegen die jeweilige spezifische Oberfläche von erfindungsgemäßen Chromatographiemedien.

**Patentansprüche**

**1.** Chromatographiemedium, umfassend

eine poröse Matrix; und
unporöse Mikroglobuli,
wobei die unporösen Mikroglobuli auf den inneren und äußeren Oberflächen der porösen Matrix kovalent gebunden sind,
wobei der durchschnittliche Radius der Mikroglobuli nicht mehr als 30% des durchschnittlichen Porendurchmessers der porösen Matrix beträgt, wobei der Porendurchmesser der porösen Matrix und der Radius der Mikroglobuli über Rasterelektronenmikroskopie bestimmt werden;
wobei der Pfropfgrad P des Chromatographiemediums von 25% bis 40% beträgt, wobei gilt:

$$P = \frac{m_m - m_0}{m_0} * 100\%$$

wobei $m_m$ die Masse des Chromatographiemediums ist und mo die Masse der porösen Matrix ohne Mikroglobuli ist, und
wobei der relative Pfropfgrad $P_{rel}$ des Chromatographiemediums höchstens 0,25 g/cm$^3$ beträgt, wobei gilt:

$$P_{rel} = \frac{P * \rho}{Por}$$

wobei P der Pfropfgrad des Chromatographiemediums in % ist, ρ die Dichte der porösen Matrix ohne Mikroglobuli in g/cm$^3$ ist, und Por die Porosität der porösen Matrix ohne Mikroglobuli in % ist,
wobei die unporösen Mikroglobuli im Wesentlichen kugelförmige Oligomere
und/oder Polymere sind, die aus mindestens einem Monomer, ausgewählt aus der Gruppe, bestehend aus Glycidyl(meth)acrylat, substituierten oder unsubstituierten Alkyl(meth)acrylaten und ihren Derivaten, Styrol und seinen Derivaten, 2-Vinyl-4,4-dimethylazlacton, substituierten oder unsubstituierten N-Alkyl(meth)acrylamiden und ihren Derivaten und substituierten oder unsubstituierten N,N'-Dialkyl(meth)acrylamiden und ihren Derivaten, aufgebaut sind, und
wobei die Oligomere und/oder Polymere miteinander und mit den inneren und äußeren Oberflächen der porösen Matrix über einen oder mehrere Vernetzer, ausgewählt aus der Gruppe, bestehend aus Ethylenglycoldimethacrylat, Trimethylpropantrimethacrylat, Divinylbenzol und N,N'-Methylenbisacrylamid, vernetzt sind.

**2.** Chromatographiemedium nach Anspruch 1, wobei die Permeabilität des Chromatographiemediums mindestens

40% der Permeabilität der porösen Matrix ohne gebundene Mikroglobuli beträgt.

3. Chromatographiemedium nach Anspruch 1 oder 2, wobei die unporösen Mikroglobuli zusätzliche chromatographisch aktive Zentren bzw. Liganden aufweisen, die an die Mikroglobuli gebunden bzw. auf diesen immobilisiert sind.

4. Verfahren zur Herstellung eines Chromatographiemediums nach einem der Ansprüche 1 bis 3, umfassend

Bereitstellen einer porösen Ausgangsmatrix, wobei die poröse Ausgangsmatrix originär zur radikalischen Polymerisation befähigte Gruppen aufweist, oder die poröse Ausgangsmatrix eine Matrix ist, bei der durch Oberflächenmodifikation funktionelle Gruppen eingeführt wurden; Bereitstellen einer Polymerisationslösung, umfassend mindestens ein Monomer, einen bi-, tri- oder multifunktionellen Vernetzer, einen Polymerisationsinitiator und ein Lösungsmittel oder Lösungsmittelgemisch, wobei das mindestens eine Monomer, der bi-, tri- oder multifunktionelle Vernetzer und der Polymerisationsinitiator in dem Lösungsmittel oder Lösungsmittelgemisch vollständig löslich sind; und
Initiieren einer Polymerisation in der Polymerisationslösung in Anwesenheit der porösen Ausgangsmatrix zur Bildung von unporösen Mikroglobuli, wobei die unporösen Mikroglobuli in dem Lösungsmittel oder Lösungsmittelgemisch unlöslich sind und an die inneren und äußeren Oberflächen der porösen Ausgangsmatrix kovalent gebunden werden;
wobei der durchschnittliche Radius der Mikroglobuli nicht mehr als 30% des durchschnittlichen Porendurchmessers der porösen Ausgangsmatrix beträgt, wobei der Radius der Poren der porösen Matrix und der Radius der Mikroglobuli über Rasterelektronenmikroskopie bestimmt werden;
wobei das Gesamtvolumen von Monomer und Vernetzer, bezogen auf das Gesamtvolumen der Polymerisationslösung, maximal 20 Vol.-% beträgt; wobei das Gesamtvolumen des Vernetzers, bezogen auf das Gesamtvolumen der Polymerisationslösung, maximal 6 Vol.-% beträgt;
wobei das mindestens eine Monomer ausgewählt ist aus der Gruppe, bestehend aus Glycidyl(meth)acrylat, substituierten oder unsubstituierten Alkyl(meth)acrylaten und ihren Derivaten, Styrol und seinen Derivaten, 2-Vinyl-4,4-dimethylazlacton, substituierten oder unsubstituierten N-Alkyl(meth)acrylamiden und ihren Derivaten und substituierten oder unsubstituierten N,N'-Dialkyl(meth)acrylamiden und ihren Derivaten;
wobei der bi-, tri- oder multifunktionelle Vernetzer ausgewählt ist aus der Gruppe, bestehend aus Ethylenglycoldimethacrylat, Trimethylpropantrimethacrylat, Divinylbenzol und N,N'-Methylenbisacrylamid; wobei der Polymerisationsinitiator ausgewählt ist aus der Gruppe, bestehend aus 2-Hydroxy-4-(2-hydroxyethoxy)-2-methylpropiophenon, Azobis(isobutyronitril), 4,4'-Azobis(4-cyanovaleriansäure), 1,1'-Azobis(cyclohexancarbonitril), Benzoylperoxid, 2,2-Bis(tert-butylperoxy)butan, 1,1-Bis(tert-butylperoxy)cyclohexan, tert-Butylhydroperoxid, tert-Butylperoxyisopropylcarbonat, Cyclohexanonperoxid und 2,4-Pentandionperoxid;
wobei das Lösungsmittel oder Lösungsmittelgemisch ausgewählt ist aus der Gruppe, bestehend aus Cyclohexanol/Dodecan-1-ol, Octan-2-on, n-Butylacetat, p-Xylen, Toluol, Ethylacetat, Benzonitril, Cyclohexanon, Dodecan-1-ol, Acetonitril/Ethanol/Wasser, Decan-1-ol und Isopropanol/Decan-1-ol; und
wobei die Konzentration des Polymerisationsinitiators in der Polymerisationslösung von 1 bis 3 Gew.-% beträgt.

5. Verfahren zur Herstellung eines Chromatographiemediums nach Anspruch 4, wobei eine makroskopisch beobachtbare Phasenseparation nach maximal 30 Sekunden eintritt.

## Claims

1. A chromatography medium, comprising

a porous matrix; and
non-porous microglobules,
wherein the non-porous microglobules are covalently bonded on the inner and outer surfaces of the porous matrix,
wherein the average radius of the microbglobules is not more than 30% of the average pore diameter of the porous matrix, wherein the pore diameter of the porous matrix and the radius of the microglobules is determined via scanning electron microscopy;
wherein the grafting degree P of the chromatography medium is from 25% to 40%,
wherein:

$$P = \frac{m_m - m_0}{m_0} * 100\%$$

wherein $m_m$ is the mass of the chromatography medium and $m_0$ is the mass of the porous matrix without microglobules, and
wherein the relative grafting degree $P_{rel}$ of the chromatography medium is not more than 0.25 g/cm$^3$, wherein:

$$P_{rel} = \frac{P * \rho}{Por}$$

wherein P is the grafting degree of the chromatography medium in %, ρ is the density of the porous matrix without microglobules in g/cm$^3$, and Por is the porosity of the porous matrix without microglobules in %,
wherein the non-porous microglobules are substantially spherical oligomers and/or polymers, which are constructed of at least one monomer, selected from the group consisting of glycidyl(meth)acrylate, substitued or unsubstituted alkyl(meth)acrylates and their derivatives, styrene and its derivatives, 2-vinyl-4,4-dimethylazlacton, substituted or unsubstituted N-alkyl(meth)acrylamides and their derivatives and substituted or unsubstituted N,N'-dialkyl(meth)acrylamides and their derivatives, and
wherein the oligomers and/or polymers are crosslinked with one another and with the inner and outer surfaces of the porous matrix via one or more crosslinker, selected from the group consisting of ethylene glycol dimethacrylate, trimethylpropane trimethacrylate, divinylbenzene and N,N'-methylenebisacrylamide.

**2.** The chromatography medium according to claim 1, wherein the permeability of the chromatography medium is at least 40% of the permeability of the porous matrix without bonded microglobules.

**3.** The chromatography medium according to claim 1 or 2, wherein the non-porous microglobules have additional chromatographically active centers or ligands, which are bound to the microglobules or are immobilized on these.

**4.** A method for producing a chromatography medium according to any of claims 1 to 3, comprising

providing a porous starting matrix, wherein the porous starting matrix has groups originally capable of radical polymerization, or the porous starting matrix is a matrix in which functional groups are introduced by means of surface modification;
providing a polymerization solution, comprising at least one monomer, a bi-, tri- or multifunctional crosslinker, a polymerization initiator and a solvent or solvent mixture, wherein the at least one monomer, the bi-, tri- or multifunctional crosslinker and the polymerization initiator are completely soluble in the solvent or solvent mixture; and
initiating a polymerization in the polymerization solution in the presence of the porous starting matrix to form non-porous microglobules, wherein the non-porous microglobules are insoluble in the solvent or solvent mixture and are covalently bonded to the inner and outer surfaces of the porous starting matrix;
wherein the average radius of the microglobules is not more than 30% of the average pore diameter of the porous starting matrix,
wherein the radius of the pores of the porous matrix and the radius of the microglobules are determined via scanning electron microscopy;
wherein the total volume of the monomer and crosslinker, with reference to the total volume of the polymerization solution is not more than 20 Vol.-%;
wherein the total volume of the crosslinker, with reference to the total volume of the polymerization solution is not more than 6 Vol.-%;
wherein the at least one monomer is selected from the group consisting of glycidyl(meth)acrylate, substituted or unsubstituted alkyl(meth)acrylates and their derivatives, styrene and its derivatives, 2-vinyl-4,4-dimethylazlactone, substituted or unsubstituted N-alkyl(meth)acrylamides and their derivatives an substituted or unsubstituted N,N'-dialkyl(meth)acrylamides and their derivatives;
wherein the bi-, tri- or multifunctional crosslinker is selected from the group consisting of ethylene glycoldimethacrylate, trimethylpropane trimethacrylate, divinylbenzene and N,N'methylenebisacrylamide;
wherein the polymerization initiator is selected from the group consisting of 2-hydroxy-4(2-hydroxyethoxy)-2-methylpropiophenone, azobis(isobutyronitrile), 4,4'-azobis(4-cyanovaleric acid), 1,1'-azobis(cyclohexanecarbonitrile), benzoyl peroxide, 2,2'Bis(tert-butylperoxy)butane, 1,1-Bis(tert-butylperoxy)cyclohexane, tert-Butyl

hydroperoxide, tert-Butylperoxy isopropyl carbonate, cyclohexane peroxide and 2,4-Pentanedione peroxide; wherein the solvent or solvent mixture is selected from the group consisting of cyclohexanol/dodecan-1-ol, octane-2-on, n-butyl acetate, p-xylene, toluene, ethyl acetate, benzonitrile, cyclohexanone, dodecan-1-ol, acetonitrile/ethanol/water, decan-1-o and isopropanol/decan-1-ol; and wherein the concentration of the polymerization initiator in the polymerization solution is from 1 to 3% by weight.

**5.** The method for producing a chromatography medium according to claim 4, wherein a macroscopically observable phase separation occurs after not more than 30 seconds.

**Revendications**

**1.** Milieu de chromatographie, comprenant

une matrice poreuse ; et
des microglobules non poreux,
dans lequel les microglobules non poreux sont liés par covalence sur les surfaces intérieures et extérieures de la matrice poreuse,
dans lequel le rayon moyen des microglobules ne s'élève pas à plus de 30 % du diamètre de pore moyen de la matrice poreuse, dans lequel le diamètre de pore de la matrice poreuse et le rayon des microglobules sont déterminés via une microscopie électronique à balayage ;
dans lequel le taux de greffage P du milieu de chromatographie s'élève de 25 % à 40 %, où :

$$P = \frac{m_m - m_0}{m_0} * 100\%$$

où $m_m$ est la masse du milieu de chromatographie et $m_0$ est la masse de la matrice poreuse sans microglobules, et où le taux de greffage relatif $P_{rel}$ du milieu de chromatographie s'élève au plus à 0,25 g/cm$^3$, où :

$$P_{rel} = \frac{P * \rho}{Por}$$

où P est le taux de greffage du milieu de chromatographie en %, p est l'épaisseur de la matrice poreuse sans microglobules en g/cm$^3$, et Por est la porosité de la matrice poreuse sans microglobules en %,
dans lequel les microglobules non poreux sont essentiellement des oligomères et/ou des polymères sphériques qui sont constitués d'au moins un monomère sélectionné dans le groupe constitué d'un (méth)acrylate de glycidyle, des (méth)acrylates d'alkyle substitués ou non substitués et leurs dérivés, un styrène et ses dérivés, une 2-vinyle-4,4-diméthylazlactone, des N-(méth)acrylamides d'alkyle substitués ou non substitués et leurs dérivés, et des N,N'-(méth)acrylamides de dialkyle substitués ou non substitués et leurs dérivés, et
dans lequel les oligomères et/ou polymères sont réticulés ensemble et avec les surfaces intérieures et extérieures de la matrice poreuse via un ou plusieurs agents de réticulation sélectionnés dans le groupe constitué d'un diméthacrylate d'éthylène glycol, un triméthacrylate de triméthylpropane, un divinylbenzol et un N,N'-bisacrylamide de méthylène.

**2.** Milieu de chromatographie selon la revendication 1, dans lequel la perméabilité du milieu de chromatographie s'élève au moins à 40 % de la perméabilité de la matrice poreuse sans microglobules liés.

**3.** Milieu de chromatographie selon la revendication 1 ou 2, dans lequel les microglobules non poreux présentent des centres ou des ligands complémentaires chromatographiquement actifs qui sont liés aux microglobules ou immobilisés sur ceuxci.

**4.** Procédé de fabrication d'un milieu de chromatographie selon l'une des revendications 1 à 3, comprenant

la fourniture d'une matrice de départ poreuse, dans lequel la matrice de départ poreuse présente des groupes à l'origine capables de polymérisation radicalaire, ou la matrice de départ poreuse est une matrice pour laquelle des groupes fonctionnels sont introduits par modification de surface ;

la fourniture d'une solution de polymérisation, qui comprend au moins un monomère, un agent de réticulation bi, tri ou multifonctionnel, un amorceur de polymérisation et un solvant ou un mélange de solvants, dans lequel le au moins un monomère, l'agent de réticulation bi, tri ou multifonctionnel et l'amorceur de polymérisation sont entièrement solubles dans le solvant ou le mélange de solvants ; et

l'amorce d'une polymérisation dans la solution de polymérisation en présence de la matrice de départ poreuse pour former des microglobules non poreux, dans lequel les microglobules non poreux ne sont pas solubles dans le solvant ou le mélange de solvants et se voient liés par covalence aux surfaces intérieures et extérieures de la matrice de départ poreuse ;

dans lequel le rayon moyen des microglobules ne s'élève pas à plus de 30 % du diamètre de pore moyen de la matrice de départ poreuse,

dans lequel le rayon des pores de la matrice poreuse et le rayon des microglobules sont déterminés via une microscopie électronique à balayage ;

dans lequel le volume global de monomères et d'agents de réticulation, relativement au volume global de la solution de polymérisation, s'élève au maximum à 20 % en volume ;

dans lequel le volume global de l'agent de réticulation, relativement au volume global de la solution de polymérisation, s'élève au maximum à 6 % en volume ;

dans lequel le au moins un monomère est sélectionné dans le groupe constitué d'un (méth)acrylate de glycidyle, des (méth)acrylates d'alkyle substitués ou non substitués et leurs dérivés, un styrène et ses dérivés, une 2-vinyle-4,4-diméthylazlactone, des N-(méth)acrylamides d'alkyle substitués ou non substitués et leurs dérivés, et des N,N'-(méth)acrylamides de dialkyle substitués ou non substitués et leurs dérivés, et

dans lequel l'agent de réticulation bi, tri ou multifonctionnel est sélectionné dans le groupe constitué d'un diméthacrylate d'éthylène glycol, un triméthacrylate de triméthylpropane, un divinylbenzol et un N,N'-bisacrylamide de méthylène ;

dans lequel l'amorceur de polymérisation est sélectionné dans le groupe constitué d'un 2-hydroxy-4-(2-hydroxyéthoxy)-2-méthylpropiophénone, un azobis(isobutyronitrile), un 4,4'-azobis(4-acide cyanovalérique), un 1-1'-azobis(carbonitrile de cyclohexane), un peroxyde de benzoyle, un 2,2-bis(tert-butylperoxy)butane, un 1,1-bis(tert-butylperoxy)cyclohexane, un hydroperoxyde de tert-butyle, un carbonate d'isopropyle et de peroxy-tert-butyle, un péroxide de cyclohexane et un 2,4-péroxide de pentandione ;

dans lequel le solvant ou le mélange de solvants est sélectionné dans le groupe constitué d'un cyclohexanol/dodécane-1—ol, un octane-2-on, un n-butylacétate, un p-xylène, un toluène, un acétate d'éthyle, un benzonitrile, une cyclohexanone, un dodécane-1—ol, un acétonitrile/éthanol/eau, un décane-1—ol et un isopropanol/décane-1-ol ; et

dans lequel la concentration de l'amorceur de polymérisation dans la solution de polymérisation s'élève de 1 à 3 % en poids.

**5.** Procédé de fabrication d'un milieu de chromatographie selon la revendication 4, dans lequel une séparation de phases macroscopiquement observable survient après au maximum 30 secondes.

Figur 1

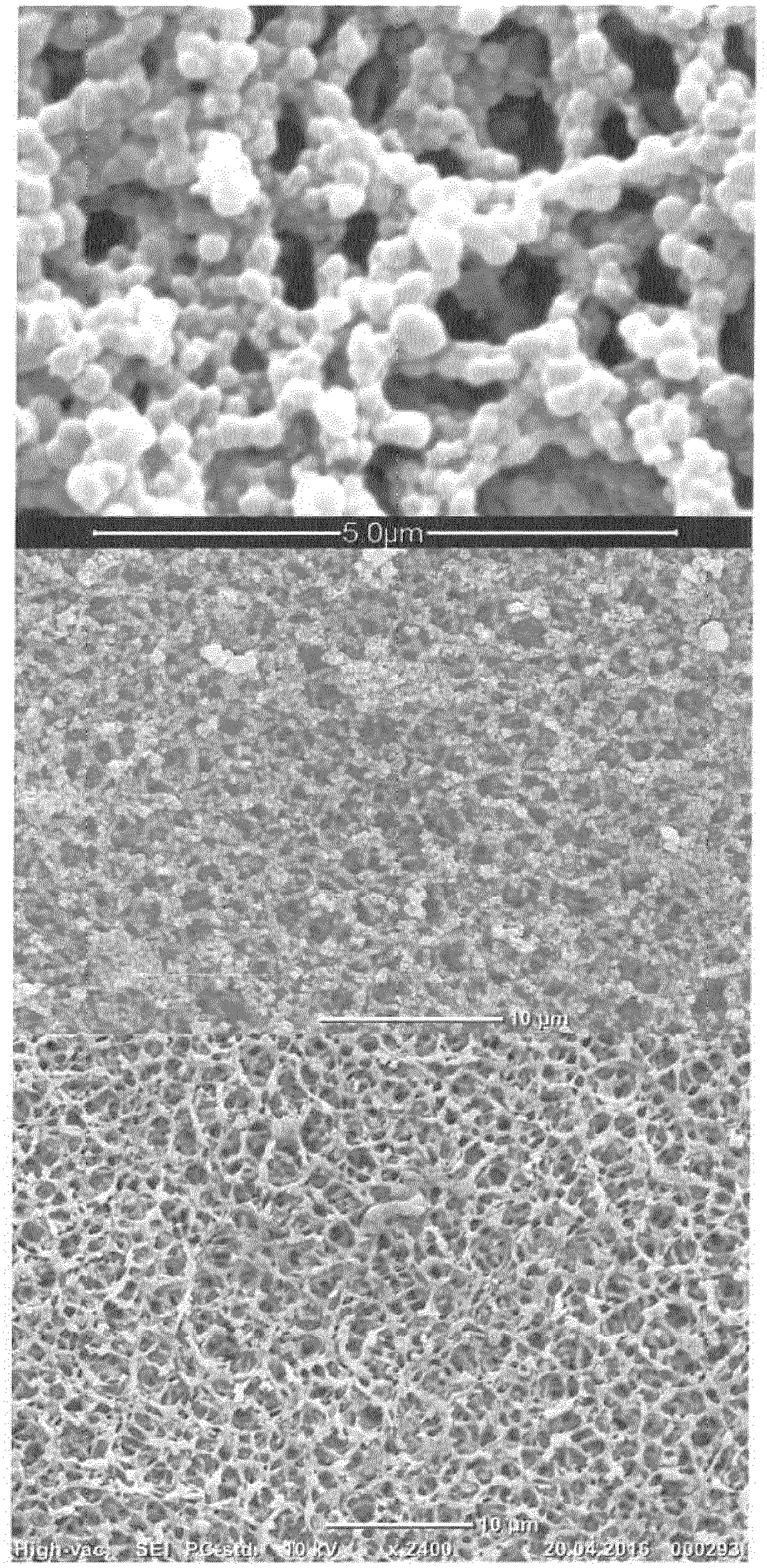
5.0µm
10 µm
10 µm
High-vac. SEI PC-std. 10 kV x 2400 20.04.2016 000293

Figur 2

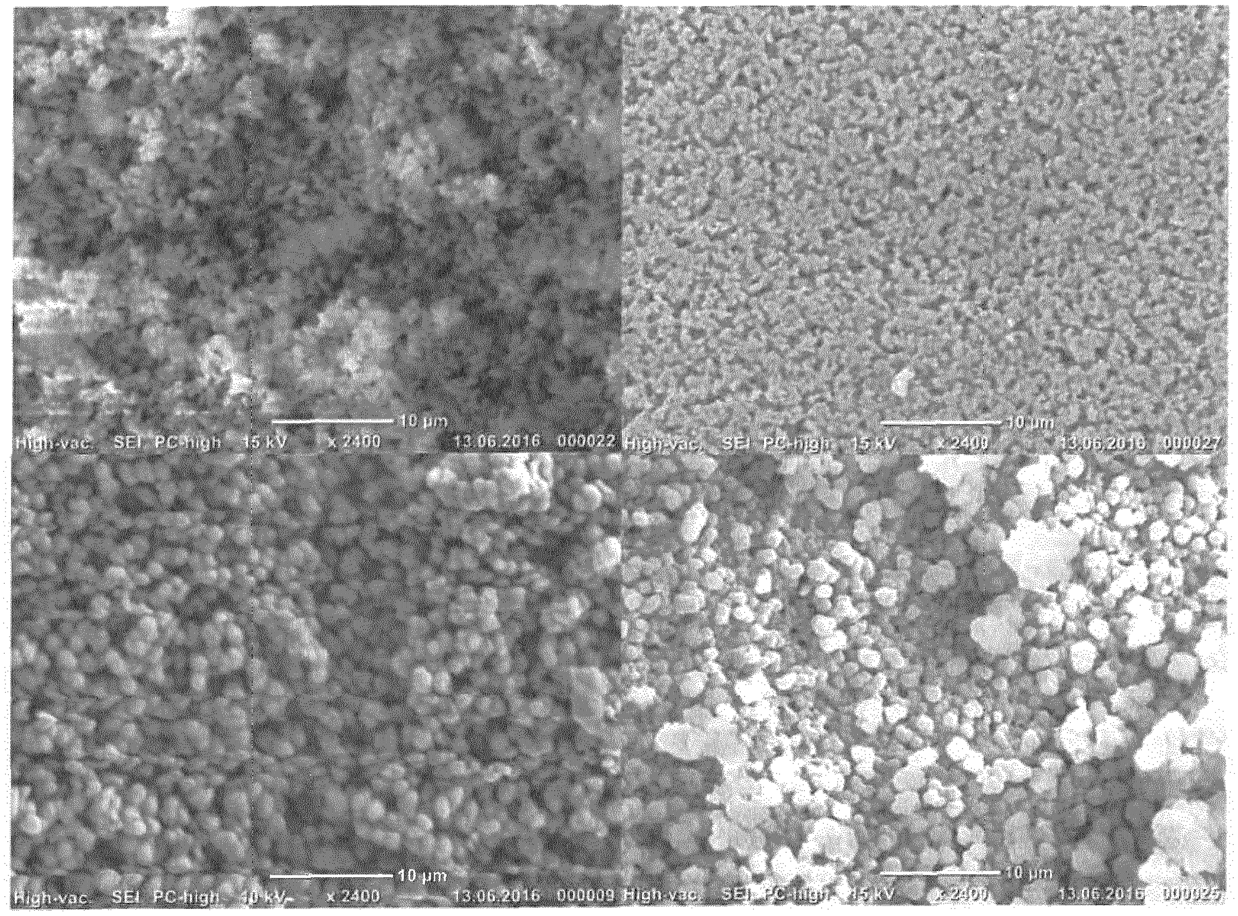
High-vac. SEI PC-high 15 kV x 2400 10 µm 13.06.2016 000022
High-vac. SEI PC-high 15 kV x 2400 10 µm 13.06.2016 000027
High-vac. SEI PC-high 10 kV x 2400 10 µm 13.06.2016 000009
High-vac. SEI PC-high 15 kV x 2400 10 µm 13.06.2016 000025

Figur 3

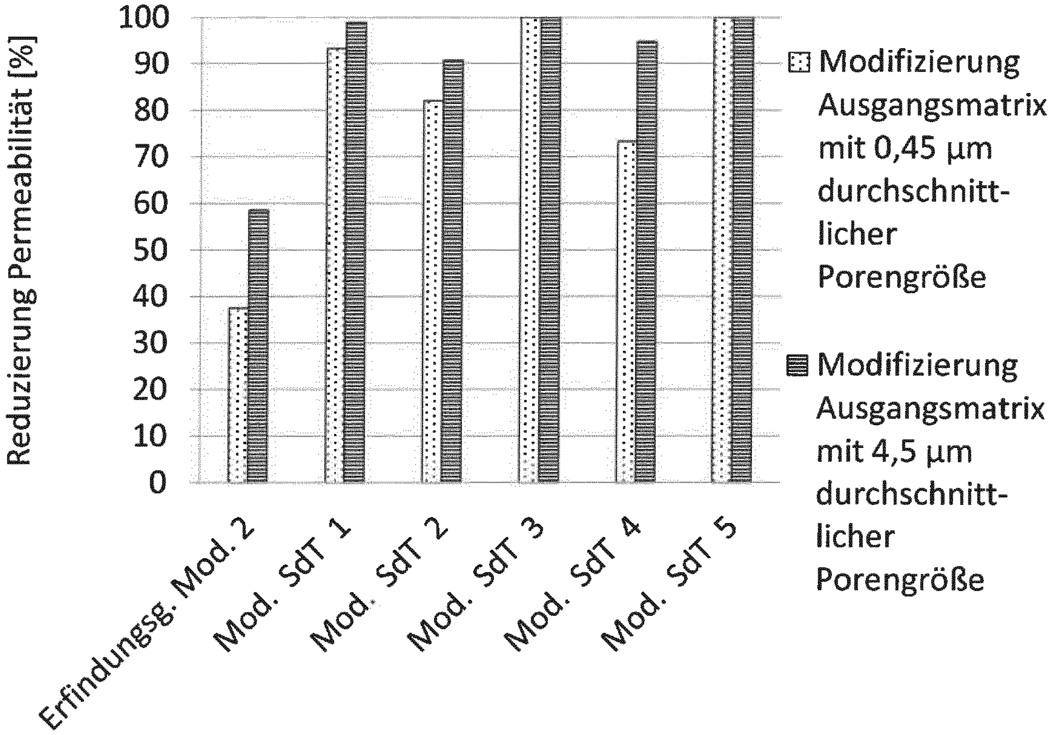


Figur 4

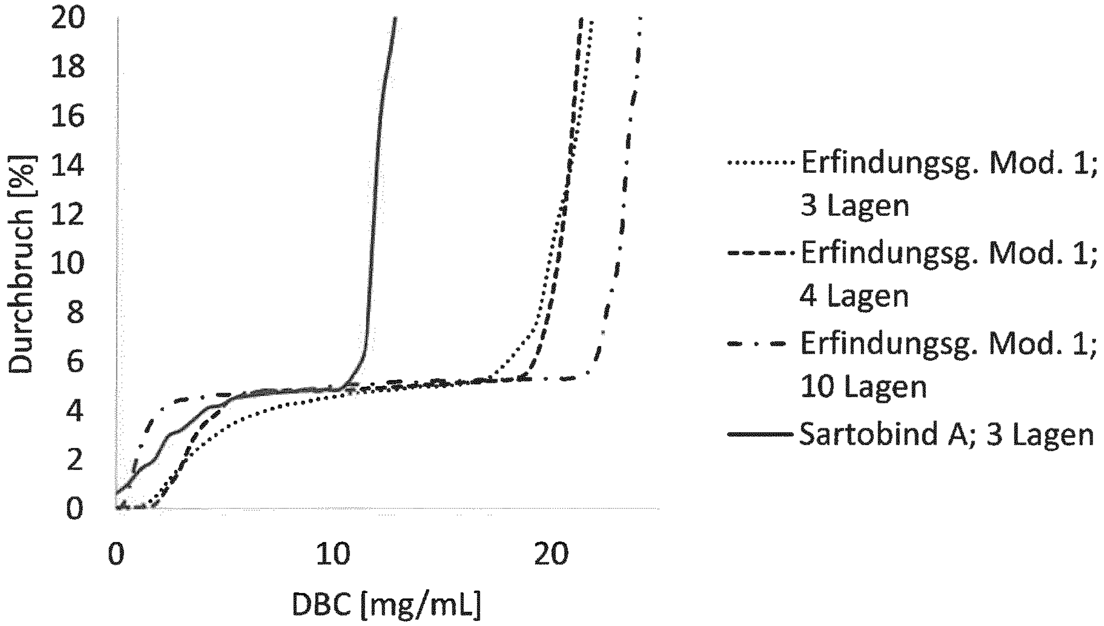

Figur 5

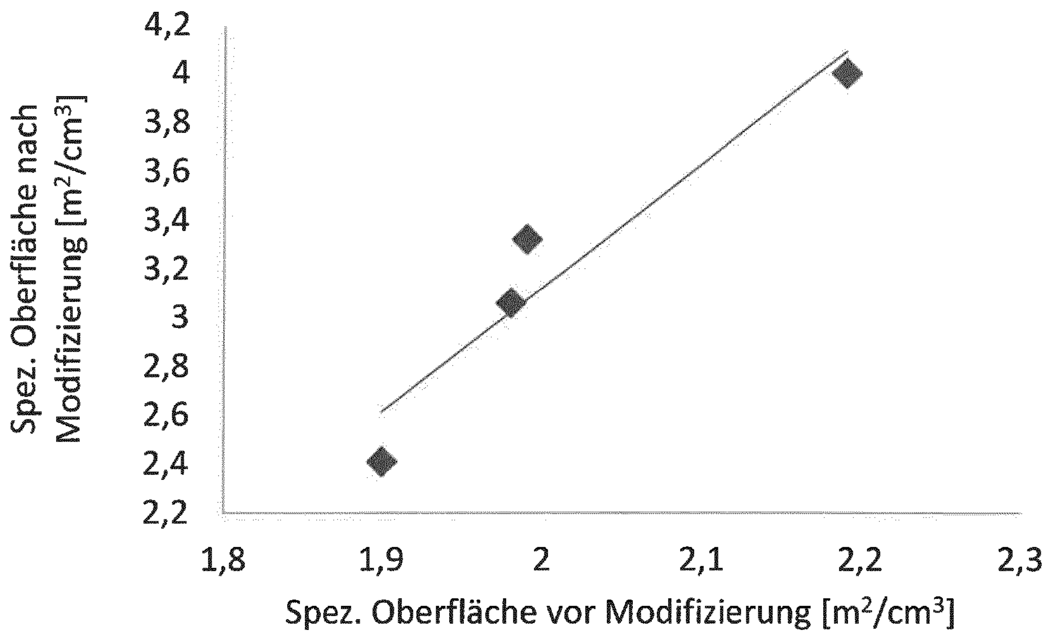


Figur 6

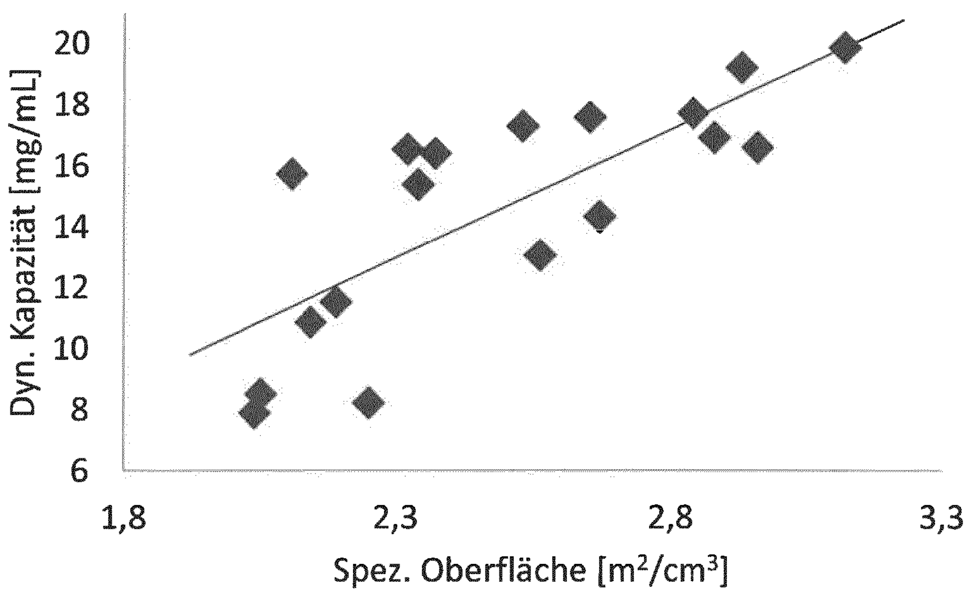

Figur 7

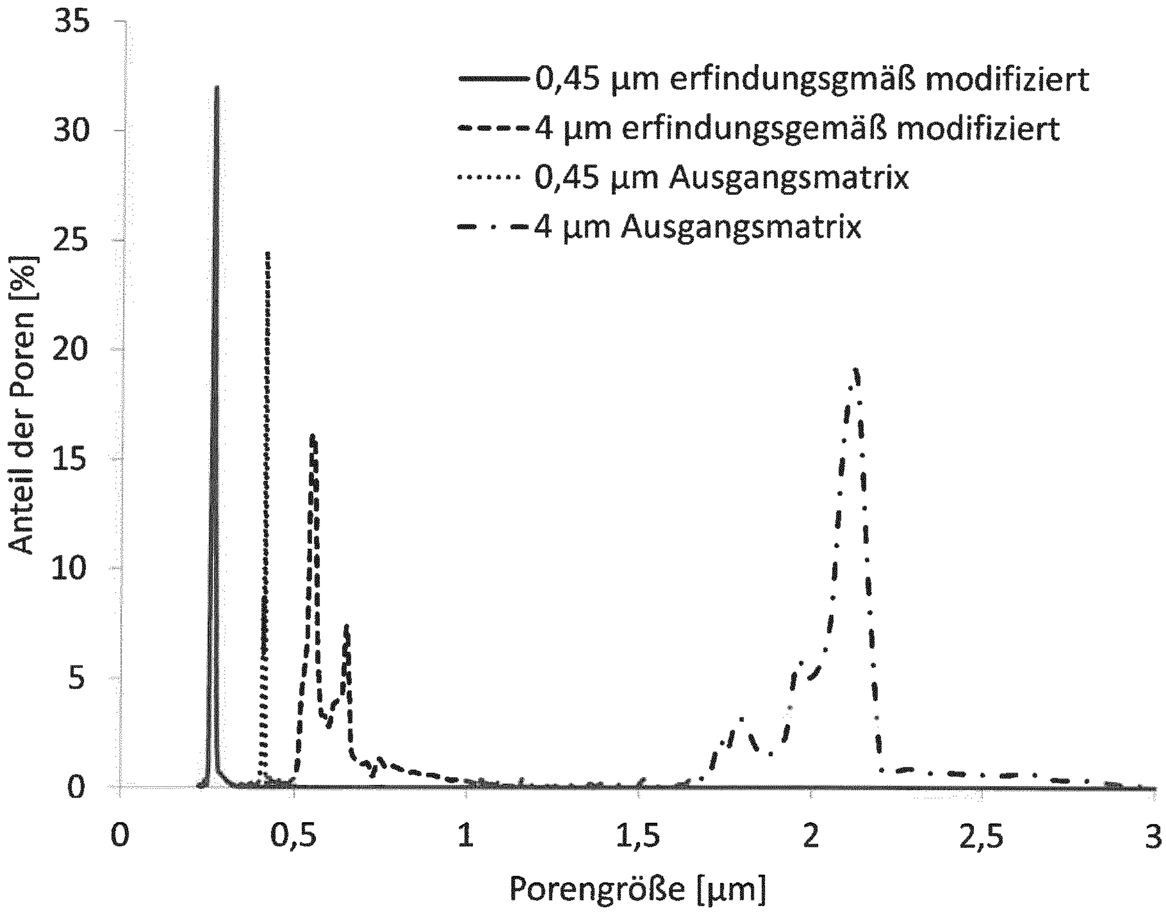
0,45 µm erfindungsgmäß modifiziert
4 µm erfindungsgemäß modifiziert
0,45 µm Ausgangsmatrix
4 µm Ausgangsmatrix
Anteil der Poren [%]
Porengröße [µm]
0
5
10
15
20
25
30
35
0,5
1
1,5
2
2,5
3

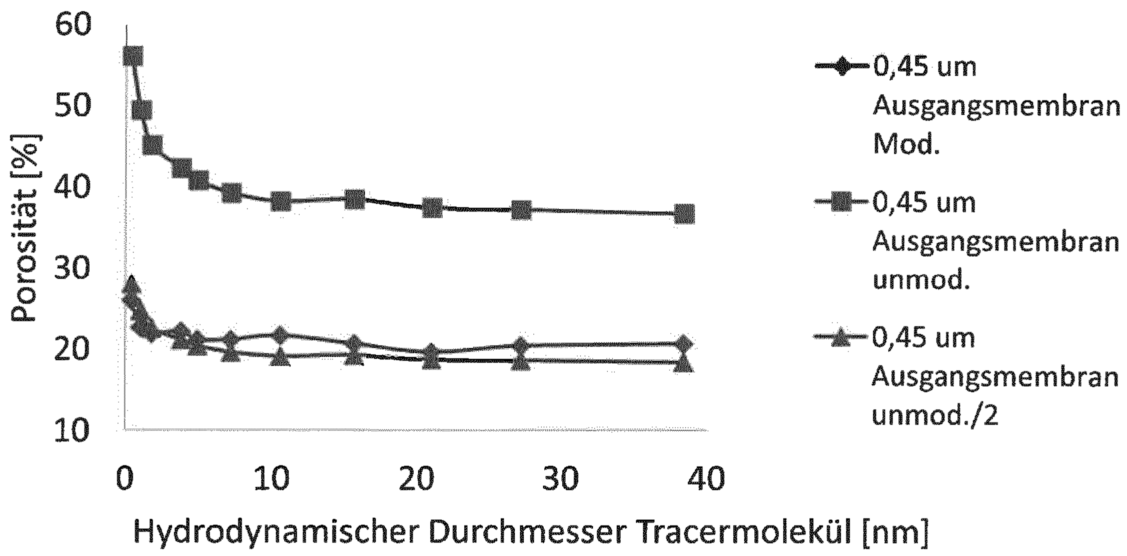
0,45 um Ausgangsmembran Mod.
0,45 um Ausgangsmembran unmod.
0,45 um Ausgangsmembran unmod./2
Porosität [%]
Hydrodynamischer Durchmesser Tracermolekül [nm]
10
20
30
40
50
60
0

Figur 8

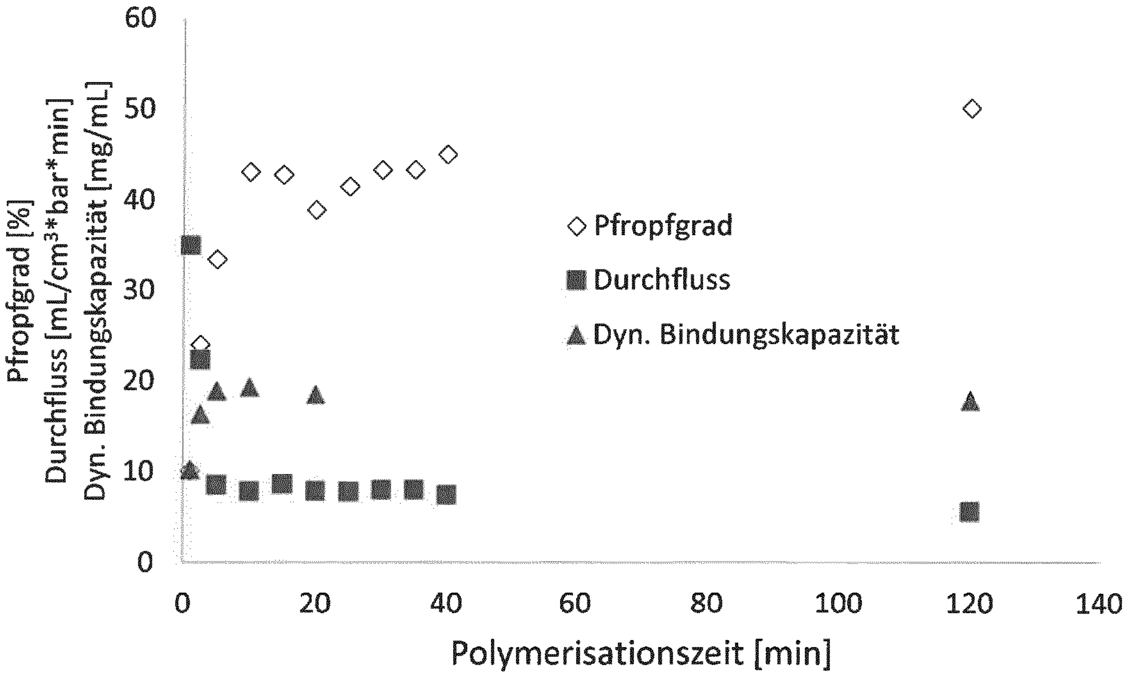


Figur 9

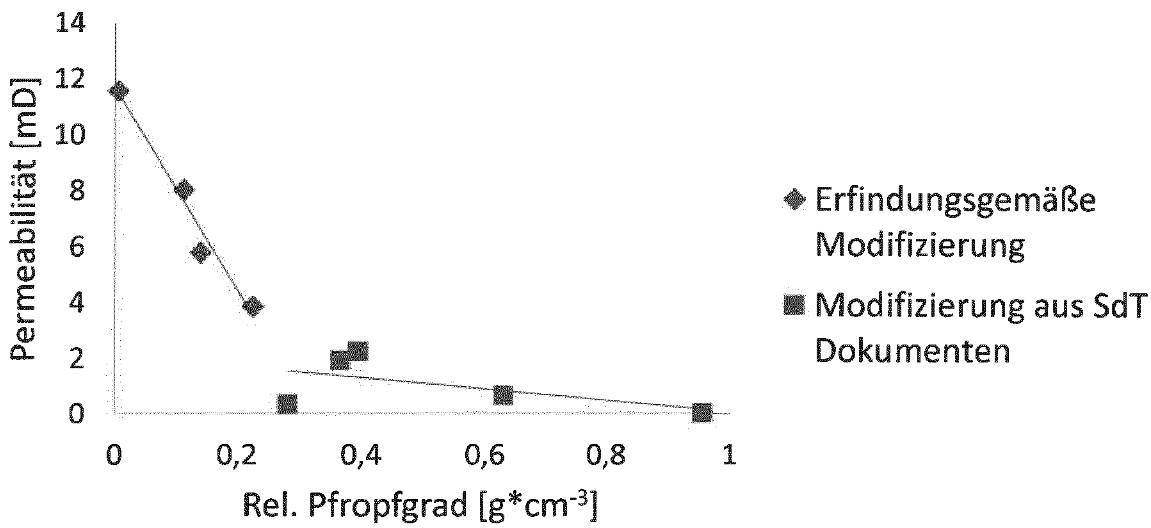

Figur 10

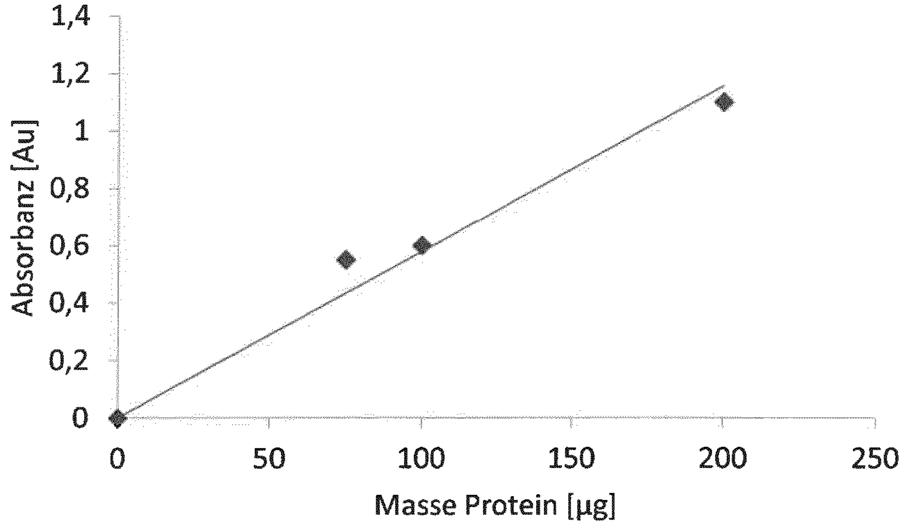


Figur 11

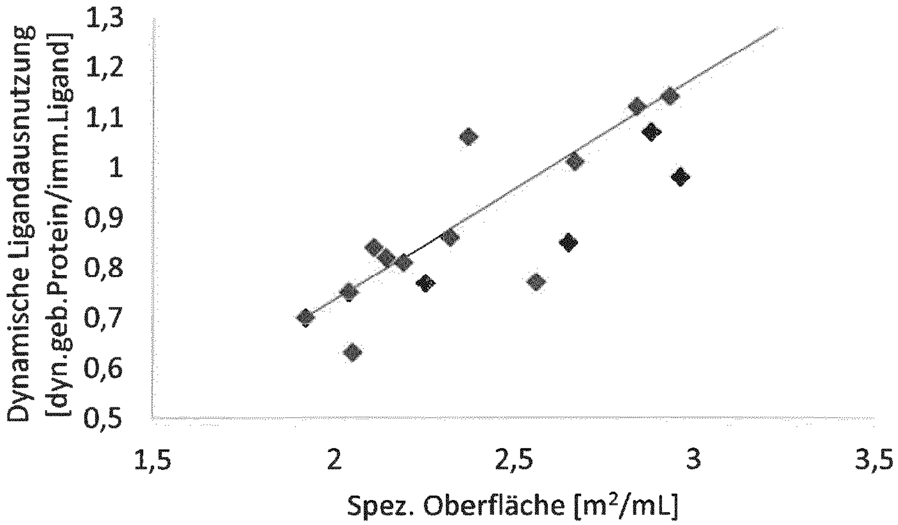

Figur 12

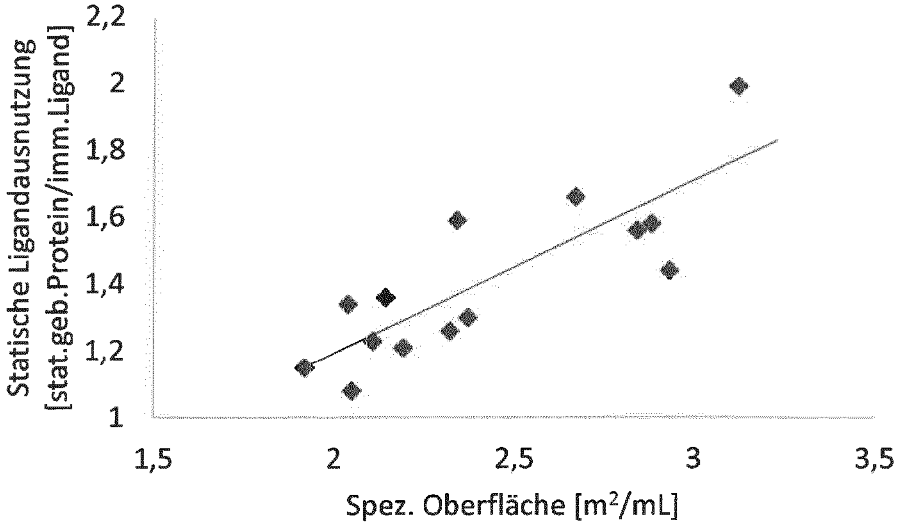


Figur 13

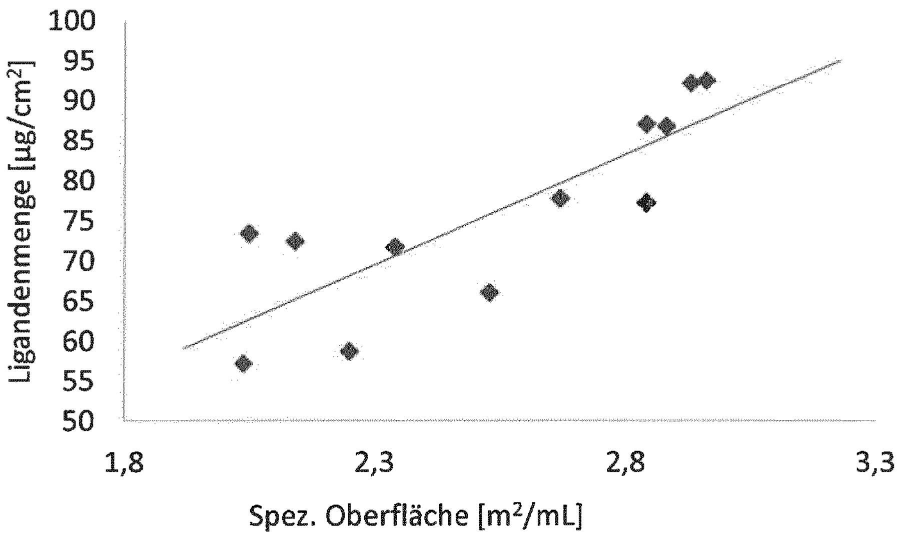

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 8652849 B2
- US 7316919 B2
- US 8133840 B2
- US 7247370 B2
- EP 1776176 B1
- WO 9325594 A1


### In der Beschreibung aufgeführte Nicht-Patentliteratur


- **KATSUYOSHI NISHINARI.** Dictionary of Polymers. *Progr. Colloid Polym. Sci.,* 2009, vol. 136, 87-94
- **KATSUYOSHI NISHINARI.** Gels: Structures, Properties, and Functions: Fundamentals and Applications. Springer Verlag Berlin Heidelberg, 2009
- **HAYNES ; SARMA et al.** *AIChE Journal,* vol. 19 (5), 1043-1046
- **SEGRE et al.** *Biomacromolecules,* 2003, vol. 4, 1843-1847